# EUROPEAN PATENT APPLICATION

(11) **EP 3 903 575 A1**
(43) Date of publication of application: **03.11.2021**
(21) Application number: 19902010.8
(22) Date of filing: 26.12.2019
(51) Int. Cl.: A01H 1/00, A01H 1/06, C12P 19/44, C12N 5/04, A23L 27/00, A23L 33/105, A61K 47/26, A61K 47/46, A01H 5/00, A01H 5/02, A01H 5/06, A01H 5/10, A01H 5/12, C12Q 1/683, C12Q 1/6844, C12Q 1/6876

(54) **LOW-TYRAMINE STEVIA PLANT**

(30) Priority: 28.12.2018 JP 2018248657
(71) Applicant: Suntory Holdings Limited, Osaka 530-8203 (JP)
(72) Inventor: HIRAI, Tadayoshi, Soraku-gun, Kyoto 619-0284 (JP); IWAKI, Kazunari, Kawasaki-shi, Kanagawa 211-0067 (JP); MIYAGAWA, Katsuro, Soraku-gun, Kyoto 619-0284 (JP)
(74) Representative: Mewburn Ellis LLP
(86) International application number: PCT/JP2019/051300
(87) International publication number: WO 2020/138365

(57) **Abstract**

The present invention provides a low tyramine-content stevia plant having a lower tyramine content as compared with the wild type stevia species. The present invention also provides a method of producing such a low tyramine-content stevia plant, and an extract and a steviol glycoside purified product obtainable from such a plant.

SEQUENCE LISTING

<110>  SUNTORY HOLDINGS LIMITED

<120>  Low Tyramine Content Stevia Plant

<130>  G2209WO

<150>  JP 2018-248657
<151>  2018-12-28

<160>  167

<170>  PatentIn version 3.5

<210>  1
<211>  399
<212>  DNA
<213>  Stevia rebaudiana

<400>  1
agactcgatc gcagcctcga tcggcgagtc ggcgattgag atgatgattc attcaacaaa      60

cacatggttt gggaatagct ctgttgttgc ctcggtcggc gaatctacaa acaagagggt     120

cttcgcttcc atgtcgaacc atttgaaaac caaagctctg aataccaaat gatgcagtta     180

atgaatacaa ccaaatggct cagaacaact gattaatcaa actcttaaaa ggaaccaaga     240

ggttcaagaa caaagttctt ataaactcaa attcaatcaa aaaactgatt tgaaacttaa     300

tttcaagtgt ttaaatagaa aacatttcta aacagataaa gactaaaatt caaataatta     360

aataaagata aactataatt tgaattaaga gatgatatg                            399


<210>  2
<211>  137
<212>  DNA
<213>  Stevia rebaudiana

<400>  2
taatcatcca aaccctaatc tcgccaaaca accgaatacc gatccaaacc ctgaaatgag      60

cacaactctt gaacctgatc acgagaatga agagcacaaa catgttatga cacatgtaaa     120

cgatggtttt tgctaca                                                    137


<210>  3
<211>  141
<212>  DNA
<213>  Stevia rebaudiana

<400>  3
aaggttcttt atttttaaac ttatgttaat ttattgtatc ttgaagttaa tcaagagatg      60

ctctcttgga gaaattttat ggtcataaaa cctatatcaa agagatgctc tcttggtata     120

ttccatactt aaaatatcta t                                               141


<210>  4

<211>  137
<212>  DNA
<213>  Stevia rebaudiana

<400>  4
cgatggtttt tgctacatga aaaccctaga agacgaaacc cgtttaagtg taaatcttga      60

aaacacattc tttgatgaag aacccctttc gtatccggat cttatggact tttctgcatc     120

gaaaaaggac gaatacg                                                    137


<210>  5
<211>  158
<212>  DNA
<213>  Stevia rebaudiana

<400>  5
cgcaaacacg tatactaatc acgtaacata ttttttattt ctaaattaaa attttataac      60

aatatcatac ttgaattaaa gataacataa tatttatttt tagagtgtaa cttctaaaaa     120

atatcaacct acgaaaaagt tgtacatacc atgctaaa                             158


<210>  6
<211>  367
<212>  DNA
<213>  Stevia rebaudiana

<400>  6
ttatttaatg atccaatgga gggggtgatt caggtaataa aaggcattcg tatggaatat      60

accaaaacat tgcgattcgt tattagcatg gatctttcaa gtaataaact tatcggagaa     120

ataccagttg agttaactgc ccttcatgcc ttggtgagtc tcaatttgtc taataatcat     180

cttattggac acattccgaa tagcattgga aacatgaaag ctttaaattc tctagatttc     240

tcgagaaacg agttaaatgg gttgatccct ccaagcattg gagctttgaa ttttttgagt    300

catttaaatt tgtcaaacaa caacttatca ggaccaattc caatcggaaa tcaattgaga     360

accctca                                                               367


<210>  7
<211>  14
<212>  DNA
<213>  Artificial Sequence

<220>
<223>  Synthetic DNA

<400>  7
atggtttggg aata                                                        14


<210>  8
<211>  15
<212>  DNA
<213>  Artificial Sequence

<220>
<223>  Synthetic DNA

<400>  8
agaactttgt tcttg                                                        15


<210>  9
<211>  15
<212>  DNA
<213>  Artificial Sequence

<220>
<223>  Synthetic DNA

<400>  9
taatcatcca aaccc                                                        15


<210>  10
<211>  15
<212>  DNA
<213>  Artificial Sequence

<220>
<223>  Synthetic DNA

<400>  10
tgtagcaaaa accat                                                        15


<210>  11
<211>  15
<212>  DNA
<213>  Artificial Sequence

<220>
<223>  Synthetic DNA

<400>  11
aaggttcttt atttt                                                        15


<210>  12
<211>  15
<212>  DNA
<213>  Artificial Sequence

<220>
<223>  Synthetic DNA

<400>  12
atagatattt taagt                                                        15


<210>  13
<211>  15
<212>  DNA
<213>  Artificial Sequence

<220>
<223>  Synthetic DNA

<400>  13
cgatggtttt tgcta                                                        15


<210>  14
<211>  15
<212>  DNA
<213>  Artificial Sequence

<220>
<223>  Synthetic DNA

<400>  14
cgtattcgtc ctttt                                                        15


<210>  15
<211>  15
<212>  DNA
<213>  Artificial Sequence

<220>
<223>  Synthetic DNA

<400>  15
cgcaaacacg tatac                                                        15


<210>  16
<211>  15
<212>  DNA
<213>  Artificial Sequence

<220>
<223>  Synthetic DNA

<400>  16
tttagcatgg tatgt                                                        15


<210>  17
<211>  14
<212>  DNA
<213>  Artificial Sequence

<220>
<223>  Synthetic DNA

<400>  17
ttatttaatg atcc                                                         14


<210>  18
<211>  15
<212>  DNA
<213>  Artificial Sequence

<220>
<223>  Synthetic DNA

<400>  18
tgagggttct caatt                                                        15

<210>  19
<211>  21
<212>  DNA
<213>  Stevia rebaudiana

<400>  19
ccaaatggct aagaacaact g                                                 21


<210>  20
<211>  41
<212>  DNA
<213>  Stevia rebaudiana

<400>  20
atgaatacaa ccaaatggct aagaacaact gattaatcaa a                           41


<210>  21
<211>  61
<212>  DNA
<213>  Stevia rebaudiana

<400>  21
gatgcagtta atgaatacaa ccaaatggct aagaacaact gattaatcaa actcttaaaa      60

g                                                                       61


<210>  22
<211>  21
<212>  DNA
<213>  Stevia rebaudiana

<400>  22
aaccgaatac tgatccaaac c                                                 21


<210>  23
<211>  41
<212>  DNA
<213>  Stevia rebaudiana

<400>  23
ctcgccaaac aaccgaatac tgatccaaac cctgaaatga g                           41


<210>  24
<211>  61
<212>  DNA
<213>  Stevia rebaudiana

<400>  24
aaaccctaat ctcgccaaac aaccgaatac tgatccaaac cctgaaatga gcacaactct      60

t                                                                       61


<210>  25
<211>  21
<212>  DNA
<213>  Stevia rebaudiana

<400>  25
ttgtatcttg tagttaatca a                                                21


<210>  26
<211>  41
<212>  DNA
<213>  Stevia rebaudiana

<400>  26
tgttaattta ttgtatcttg tagttaatca agagatgctc t                          41


<210>  27
<211>  61
<212>  DNA
<213>  Stevia rebaudiana

<400>  27
tttaaactta tgttaattta ttgtatcttg tagttaatca agagatgctc tcttggagaa      60

a                                                                      61


<210>  28
<211>  21
<212>  DNA
<213>  Stevia rebaudiana

<400>  28
acccgtttaa ctgtaaatct t                                                21


<210>  29
<211>  41
<212>  DNA
<213>  Stevia rebaudiana

<400>  29
agaagacgaa acccgtttaa ctgtaaatct tgaaaacaca t                          41


<210>  30
<211>  61
<212>  DNA
<213>  Stevia rebaudiana

<400>  30
tgaaaaccct agaagacgaa acccgtttaa ctgtaaatct tgaaaacaca ttctttgatg      60

a                                                                      61


<210>  31
<211>  20
<212>  DNA
<213>  Stevia rebaudiana

<400>  31
attaaaattt gaattaaaga                                                  20

<210>  32
<211>  40
<212>  DNA
<213>  Stevia rebaudiana

<400>  32
ttatttctaa attaaaattt gaattaaaga taacataata                              40


<210>  33
<211>  60
<212>  DNA
<213>  Stevia rebaudiana

<400>  33
aacatatttt ttatttctaa attaaaattt gaattaaaga taacataata tttattttta        60


<210>  34
<211>  21
<212>  DNA
<213>  Stevia rebaudiana

<400>  34
aaaaggcatt agtatggaat a                                                  21


<210>  35
<211>  41
<212>  DNA
<213>  Stevia rebaudiana

<400>  35
ttcaggtaat aaaaggcatt agtatggaat ataccaaaac a                            41


<210>  36
<211>  61
<212>  DNA
<213>  Stevia rebaudiana

<400>  36
gaggggtgat ttcaggtaat aaaaggcatt agtatggaat ataccaaaac attgcgattc        60

g                                                                        61


<210>  37
<211>  25
<212>  DNA
<213>  Artificial Sequence

<220>
<223>  Synthetic DNA

<400>  37
atggtttggg aatagctctg ttgtt                                              25


<210>  38
<211>  24
<212>  DNA
<213>  Artificial Sequence

<220>
<223>  Synthetic DNA

<400>  38
agaactttgt tcttgaacct cttg                                              24


<210>  39
<211>  196
<212>  DNA
<213>  Artificial Sequence

<220>
<223>  Synthetic DNA

<400>  39
atggtttggg aatagctctg ttgttgcctc ggtcggcgaa tctacaaaca agagggtctt       60

cgcttccatg tcgaaccatt tgaaaaccaa agctctgaat accaaatgat gcagttaatg      120

aatacaacca aatggctaag aacaactgat taatcaaact cttaaaagga accaagaggt      180

tcaagaacaa agttct                                                      196


<210>  40
<211>  196
<212>  DNA
<213>  Artificial Sequence

<220>
<223>  Synthetic DNA

<400>  40
atggtttggg aatagctctg ttgttgcctc ggtcggcgaa tctacaaaca agagggtctt       60

cgcttccatg tcgaaccatt tgaaaaccaa agctctgaat accaaatgat gcagttaatg      120

aatacaacca aatggctcag aacaactgat taatcaaact cttaaaagga accaagaggt      180

tcaagaacaa agttct                                                      196


<210>  41
<211>  96
<212>  DNA
<213>  Artificial Sequence

<220>
<223>  Synthetic DNA

<400>  41
atggtttggg aatagctctg ttgttgcctc ggtcggcgaa tctacaaaca agagggtctt       60

cgcttccatg tcgaaccatt tgaaaaccaa agctct                                 96


<210>  42
<211>  100
<212>  DNA
<213>  Artificial Sequence

<220>
<223>  Synthetic DNA

<400>  42
gaataccaaa tgatgcagtt aatgaataca accaaatggc taagaacaac tgattaatca      60

aactcttaaa aggaaccaag aggttcaaga acaaagttct                          100


<210>  43
<211>  43
<212>  DNA
<213>  Artificial Sequence

<220>
<223>  Synthetic DNA

<400>  43
gaataccaaa tgatgcagtt aatgaataca accaaatggc tca                       43


<210>  44
<211>  57
<212>  DNA
<213>  Artificial Sequence

<220>
<223>  Synthetic DNA

<400>  44
gaacaactga ttaatcaaac tcttaaaagg aaccaagagg ttcaagaaca aagttct        57


<210>  45
<211>  39
<212>  DNA
<213>  Artificial Sequence

<220>
<223>  Synthetic DNA

<400>  45
taatcatcca aaccctaatc tcgccaaaca accgggtac                            39


<210>  46
<211>  20
<212>  DNA
<213>  Artificial Sequence

<220>
<223>  Synthetic DNA

<400>  46
gaggaagaca ttggcaactc                                                 20


<210>  47
<211>  297
<212>  DNA
<213>  Artificial Sequence

<220>

<223>  Synthetic DNA

<400>  47
taatcatcca aaccctaatc tcgccaaaca accgggtact gatccaaacc ctgaaatgag      60

cacaactctt gaacctgatc acgagaatga agagcacaaa catgttatga cacatgtaaa     120

cgatggtttt tgctacatga aaaccctaga agacgaaacc cgtttaactg taaatcttga     180

aaacacattc tttgatgaaa aacccctttc gtatccggat cttatggact tttctgcatc     240

gaaaacggac gaatacgact tctatgatga acttgaagag ctgccaatgt cttcctc        297


<210>  48
<211>  297
<212>  DNA
<213>  Artificial Sequence

<220>
<223>  Synthetic DNA

<400>  48
taatcatcca aaccctaatc tcgccaaaca accgggtacc gatccaaacc ctgaaatgag      60

cacaactctt gaacctgatc acgagaatga agagcacaaa catgttatga cacatgtaaa     120

cgatggtttt tgctacatga aaaccctaga agacgaaacc cgtttaagtg taaatcttga     180

aaacacattc tttgatgaag aacccctttc gtatccggat cttatggact tttctgcatc     240

gaaaaaggac gaatacgact tctatgatga acttgaagag ttgccaatgt cttcctc        297


<210>  49
<211>  258
<212>  DNA
<213>  Artificial Sequence

<220>
<223>  Synthetic DNA

<400>  49
cgatccaaac cctgaaatga gcacaactct tgaacctgat cacgagaatg aagagcacaa      60

acatgttatg acacatgtaa acgatggttt ttgctacatg aaaaccctag aagacgaaac     120

ccgtttaagt gtaaatcttg aaaacacatt ctttgatgaa gaaccccttt cgtatccgga     180

tcttatggac ttttctgcat cgaaaaagga cgaatacgac ttctatgatg aacttgaaga     240

gttgccaatg tcttcctc                                                   258


<210>  50
<211>  43
<212>  DNA
<213>  Artificial Sequence

<220>
<223>  Synthetic DNA

<400>  50

aaggttcttt atttttaaac ttatgttaat ttattgtatc tag                    43

<210>  51
<211>  21
<212>  DNA
<213>  Artificial Sequence

<220>
<223>  Synthetic DNA

<400>  51
ccttatgtac acatgctaca c                                               21

<210>  52
<211>  383
<212>  DNA
<213>  Artificial Sequence

<220>
<223>  Synthetic DNA

<400>  52
aaggttcttt atttttaaac ttatgttaat ttattgtatc tagtagttaa tcaagagatg      60

ctctcttgga gaaattttat ggtcataaaa cctatatcaa agagatgctc tcttggtata     120

ttccatactt aaaatatcta ttttggaaaa aaagtgtagc atcttcctgc ttttagtagg     180

tgtcaatcat tattaaattt cacaaaaccg tgcaagaatc ccagtttccc tatagtttgt     240

atacgttcct gatctagtat tttacttatg tttcaaatca atccaatcat gcttgtgtcc     300

gaaaattaaa aaacaagggt attggatgcc ctgtaccact attattaact tttcagaaaa     360

acgtgtagca tgtgtacata agg                                             383

<210>  53
<211>  383
<212>  DNA
<213>  Artificial Sequence

<220>
<223>  Synthetic DNA

<400>  53
aaggttcttt atttttaaac ttatgttaat ttattgtatc tagaagttaa tcaagagatg      60

ctctcttgga gaaattttat ggtcataaaa cctatatcaa agagatgctc tcttggtata     120

ttccatactt aaaatatcta ttttggaaaa aaagtgtagc atcttcctgc ttttagtagg     180

tgtcaatcat tattaaattt cacaaaaccg tgcaagaatc ccagtttccc tatagtttgt     240

atacgttcct gatctagtat tttacttatg tttcaaatca gtccaatcat gcttgtgtcc     300

gaaaattaaa aaacaagggt attggatgcc ctgtaccact attattaact tttcagaaaa     360

acgtgtagca tgtgtacata agg                                             383

<210> 54
<211> 344
<212> DNA
<213> Artificial Sequence

<220>
<223> Synthetic DNA

<400> 54
ctagaagtta atcaagagat gctctcttgg agaaatttta tggtcataaa acctatatca 60

aagagatgct ctcttggtat attccatact taaaatatct attttggaaa aaaagtgtag 120

catcttcctg cttttagtag gtgtcaatca ttattaaatt tcacaaaacc gtgcaagaat 180

cccagtttcc ctatagtttg tatacgttcc tgatctagta ttttacttat gtttcaaatc 240

agtccaatca tgcttgtgtc cgaaaattaa aaaacaaggg tattggatgc cctgtaccac 300

tattattaac ttttcagaaa aacgtgtagc atgtgtacat aagg 344


<210> 55
<211> 47
<212> DNA
<213> Artificial Sequence

<220>
<223> Synthetic DNA

<400> 55
cgatggtttt tgctacatga aaaccctaga agacgaaacc cgcttaa 47


<210> 56
<211> 23
<212> DNA
<213> Artificial Sequence

<220>
<223> Synthetic DNA

<400> 56
accagcaata atccttgaat tag 23


<210> 57
<211> 390
<212> DNA
<213> Artificial Sequence

<220>
<223> Synthetic DNA

<400> 57
cgatggtttt tgctacatga aaaccctaga agacgaaacc cgcttaactg taaatcttga 60

aaacacattc tttgatgaaa aacccctttc gtatccggat cttatggact tttctgcatc 120

gaaaacggac gaatacgact tctatgatga acttgaagag ctgccaatgt cttcctcatc 180

attcaaaagc ttcatgagaa gtaatttctt tgaggaaaga gttcttgttc aaccttattg 240

attaagaatt taagggaagc agattatata tgtaattaaa ttttggtatt tatactttga 300

acttaattaa taattataat aataatccca actagaggca cttagtggag attacttata 360

tataatacta attcaaggat tattgctggt 390

<210> 58
<211> 390
<212> DNA
<213> Artificial Sequence

<220>
<223> Synthetic DNA

<400> 58
cgatggtttt tgctacatga aaaccctaga agacgaaacc cgcttaagtg taaatcttga 60

aaacacattc tttgatgaag aacccctttc gtatccggat cttatggact tttctgcatc 120

gaaaaaggac gaatacgact tctatgatga acttgaagag ttgccaatgt cttcctcatc 180

attcaaaagc ttcatgagaa gtaatttctt tgaggaaaga gttcttgttc aaccttattg 240

attaagaatt taagggaagc agattatata tgtaattaaa ttttggtatt tatactttga 300

acttaattaa taattataat aataatccca actagaggca cttagtggag attacttata 360

tataatacta attcaaggat tattgctggt 390

<210> 59
<211> 347
<212> DNA
<213> Artificial Sequence

<220>
<223> Synthetic DNA

<400> 59
ttaagtgtaa atcttgaaaa cacattcttt gatgaagaac ccctttcgta tccggatctt 60

atggactttt ctgcatcgaa aaaggacgaa tacgacttct atgatgaact tgaagagttg 120

ccaatgtctt cctcatcatt caaaagcttc atgagaagta atttctttga ggaaagagtt 180

cttgttcaac cttattgatt aagaatttaa gggaagcaga ttatatatgt aattaaattt 240

tggtatttat actttgaact taattaataa ttataataat aatcccaact agaggcactt 300

agtggagatt acttatatat aatactaatt caaggattat tgctggt 347

<210> 60
<211> 20
<212> DNA
<213> Artificial Sequence

<220>
<223> Synthetic DNA

<400> 60
cgcaaacacg tatactaatc 20

<210>  61
<211>  20
<212>  DNA
<213>  Artificial Sequence

<220>
<223>  Synthetic DNA

<400>  61
tttagcatgg tatgtacaac                                                    20


<210>  62
<211>  140
<212>  DNA
<213>  Artificial Sequence

<220>
<223>  Synthetic DNA

<400>  62
cgcaaacacg tatactaatc acgtaacata ttttttattt ctaaattaaa atttgaatta        60

aagataacat aatatttatt tttagagtgt aacttctaaa aaatatcaac ctacgaaaaa       120

gttgtacata ccatgctaaa                                                   140


<210>  63
<211>  158
<212>  DNA
<213>  Artificial Sequence

<220>
<223>  Synthetic DNA

<400>  63
cgcaaacacg tatactaatc acgtaacata tttttatttt ctaaattaaa attttataac        60

aatatcatac ttgaattaaa gataacataa tatttatttt tagagtgtaa cttctaaaaa       120

atatcaacct acgaaaaagt tgtacatacc atgctaaa                               158


<210>  64
<211>  48
<212>  DNA
<213>  Artificial Sequence

<220>
<223>  Synthetic DNA

<400>  64
ttatttaatg atccaatgga gggggtgatt caggtaataa aaggcatt                    48


<210>  65
<211>  28
<212>  DNA
<213>  Artificial Sequence

<220>
<223>  Synthetic DNA

<400>  65
tgagggttct caattgattt ccgattgg                                          28


<210>  66
<211>  367
<212>  DNA
<213>  Artificial Sequence

<220>
<223>  Synthetic DNA

<400>  66
ttatttaatg atccaatgga gggggtgatt caggtaataa aaggcactag tatggaatat       60

accaaaacat tgcgattcgt tattagcatg gatctttcaa gtaataaact tatcggagaa      120

ataccagttg agttaactgc ccttcatgcc ttggtgagtc tcaatttgtc taataatcat      180

cttattggac acattccgaa tagcattgga aacatgaaag ctttaaattc tctagatttc      240

tcgagaaacg agttaaatgg gttgatccct ccaagcattg gagctttgaa ttttttgagt       300

catttaaatt tgtcaaacaa caacttatca ggaccaattc caatcggaaa tcaattgaga      360

accctca                                                                367


<210>  67
<211>  367
<212>  DNA
<213>  Artificial Sequence

<220>
<223>  Synthetic DNA

<400>  67
ttatttaatg atccaatgga gggggtgatt caggtaataa aaggcactcg tatggaatat       60

accaaaacat tgcgattcgt tattagcatg gatctttcaa gtaataaact tatcggagaa      120

ataccagttg agttaactgc ccttcatgcc ttggtgagtc tcaatttgtc taataatcat      180

cttattggac acattccgaa tagcattgga aacatgaaag ctttaaattc tctagatttc      240

tcgagaaacg agttaaatgg gttgatccct ccaagcattg gagctttgaa ttttttgagt       300

catttaaatt tgtcaaacaa caacttatca ggaccaattc caatcggaaa tcaattgaga      360

accctca                                                                367


<210>  68
<211>  321
<212>  DNA
<213>  Artificial Sequence

<220>
<223>  Synthetic DNA

<400>  68
ctagtatgga atataccaaa acattgcgat tcgttattag catggatctt tcaagtaata      60

aacttatcgg agaaatacca gttgagttaa ctgcccttca tgccttggtg agtctcaatt     120

tgtctaataa tcatcttatt ggacacattc cgaatagcat tggaaacatg aaagctttaa     180

attctctaga tttctcgaga aacgagttaa atgggttgat ccctccaagc attggagctt     240

tgaatttttt gagtcattta aatttgtcaa acaacaactt atcaggacca attccaatcg     300

gaaatcaatt gagaaccctc a                                               321


<210>  69
<211>  399
<212>  DNA
<213>  Stevia rebaudiana

<400>  69
agactcgatc gcagcctcga tcggcgagtc ggcgattgag atgatgattc attcaacaaa      60

cacatggttt gggaatagct ctgttgttgc ctcggtcggc gaatctacaa acaagagggt     120

cttcgcttcc atgtcgaacc atttgaaaac caaagctctg aataccaaat gatgcagtta     180

atgaatacaa ccaaatggct aagaacaact gattaatcaa actcttaaaa ggaaccaaga     240

ggttcaagaa caaagttctt ataaactcaa attcaatcaa aaaactgatt tgaaacttaa     300

tttcaagtgt ttaaatagaa aacatttcta aacagataaa gactaaaatt caaataatta     360

aataaagata aactataatt tgaattaaga gatgatatg                            399


<210>  70
<211>  137
<212>  DNA
<213>  Stevia rebaudiana

<400>  70
taatcatcca aaccctaatc tcgccaaaca accgaatact gatccaaacc ctgaaatgag      60

cacaactctt gaacctgatc acgagaatga agagcacaaa catgttatga cacatgtaaa     120

cgatggtttt tgctaca                                                    137


<210>  71
<211>  141
<212>  DNA
<213>  Stevia rebaudiana

<400>  71
aaggttcttt atttttaaac ttatgttaat ttattgtatc ttgtagttaa tcaagagatg      60

ctctcttgga gaaattttat ggtcataaaa cctatatcaa agagatgctc tcttggtata     120

ttccatactt aaaatatcta t                                               141


<210>  72
<211>  137

<212>  DNA
<213>  Stevia rebaudiana

<400>  72
cgatggtttt tgctacatga aaaccctaga agacgaaacc cgtttaactg taaatcttga      60

aaacacattc tttgatgaag aacccctttc gtatccggat cttatggact tttctgcatc     120

gaaaaaggac gaatacg                                                    137


<210>  73
<211>  140
<212>  DNA
<213>  Stevia rebaudiana

<400>  73
cgcaaacacg tatactaatc acgtaacata ttttttattt ctaaattaaa atttgaatta      60

aagataacat aatatttatt tttagagtgt aacttctaaa aaatatcaac ctacgaaaaa     120

gttgtacata ccatgctaaa                                                 140


<210>  74
<211>  367
<212>  DNA
<213>  Stevia rebaudiana

<400>  74
ttatttaatg atccaatgga gggggtgatt caggtaataa aaggcattag tatggaatat      60

accaaaacat tgcgattcgt tattagcatg gatctttcaa gtaataaact tatcggagaa     120

ataccagttg agttaactgc ccttcatgcc ttggtgagtc tcaatttgtc taataatcat     180

cttattggac acattccgaa tagcattgga aacatgaaag ctttaaattc tctagatttc     240

tcgagaaacg agttaaatgg gttgatccct ccaagcattg gagctttgaa ttttttgagt     300

catttaaatt tgtcaaacaa caacttatca ggaccaattc caatcggaaa tcaattgaga     360

accctca                                                               367


<210>  75
<211>  21
<212>  DNA
<213>  Stevia rebaudiana

<400>  75
ccaaatggct cagaacaact g                                                21


<210>  76
<211>  41
<212>  DNA
<213>  Stevia rebaudiana

<400>  76
atgaatacaa ccaaatggct cagaacaact gattaatcaa a                          41

<210>  77
<211>  61
<212>  DNA
<213>  Stevia rebaudiana

<400>  77
gatgcagtta atgaatacaa ccaaatggct cagaacaact gattaatcaa actcttaaaa      60

g                                                                      61


<210>  78
<211>  15
<212>  DNA
<213>  Artificial Sequence

<220>
<223>  Synthetic DNA

<400>  78
caaacaaccg ggtac                                                       15


<210>  79
<211>  15
<212>  DNA
<213>  Artificial Sequence

<220>
<223>  Synthetic DNA

<400>  79
agacattggc aactc                                                       15


<210>  80
<211>  15
<212>  DNA
<213>  Artificial Sequence

<220>
<223>  Synthetic DNA

<400>  80
atttattgta tctag                                                       15


<210>  81
<211>  15
<212>  DNA
<213>  Artificial Sequence

<220>
<223>  Synthetic DNA

<400>  81
gtacacatgc tacac                                                       15


<210>  82
<211>  15
<212>  DNA
<213>  Artificial Sequence

<220>
<223>  Synthetic DNA

<400>  82
acgaaacccg cttaa                                                        15


<210>  83
<211>  15
<212>  DNA
<213>  Artificial Sequence

<220>
<223>  Synthetic DNA

<400>  83
taatccttga attag                                                        15


<210>  84
<211>  15
<212>  DNA
<213>  Artificial Sequence

<220>
<223>  Synthetic DNA

<400>  84
acacgtatac taatc                                                        15


<210>  85
<211>  15
<212>  DNA
<213>  Artificial Sequence

<220>
<223>  Synthetic DNA

<400>  85
catggtatgt acaac                                                        15


<210>  86
<211>  20
<212>  DNA
<213>  Artificial Sequence

<220>
<223>  Synthetic DNA

<400>  86
ttcaggtaat aaaaggcctt                                                   20


<210>  87
<211>  20
<212>  DNA
<213>  Artificial Sequence

<220>
<223>  Synthetic DNA

<400>  87
ttcaggtaat aaaaggcact                                                   20


<210>  88
<211>  20
<212>  DNA
<213>  Artificial Sequence

<220>
<223>  Synthetic DNA

<400>  88
ttcaggtaat aaaaggctta                                                   20


<210>  89
<211>  20
<212>  DNA
<213>  Artificial Sequence

<220>
<223>  Synthetic DNA

<400>  89
ttcaggtaat aaaaggcttg                                                   20


<210>  90
<211>  20
<212>  DNA
<213>  Artificial Sequence

<220>
<223>  Synthetic DNA

<400>  90
ttcaggtaat aaaaggcctc                                                   20


<210>  91
<211>  20
<212>  DNA
<213>  Artificial Sequence

<220>
<223>  Synthetic DNA

<400>  91
ttcaggtaat aaaaggcacg                                                   20


<210>  92
<211>  20
<212>  DNA
<213>  Artificial Sequence

<220>
<223>  Synthetic DNA

<400>  92
ttcaggtaat aaaagtcatg                                                   20

<210>  93
<211>  20
<212>  DNA
<213>  Artificial Sequence

<220>
<223>  Synthetic DNA

<400>  93
ttcaggtaat aaaaggctrt                                                    20


<210>  94
<211>  20
<212>  DNA
<213>  Artificial Sequence

<220>
<223>  Synthetic DNA

<400>  94
ttcaggtaat aaaaggcttr                                                    20


<210>  95
<211>  20
<212>  DNA
<213>  Artificial Sequence

<220>
<223>  Synthetic DNA

<400>  95
ttcaggtaat aaaaggcagc                                                    20


<210>  96
<211>  20
<212>  DNA
<213>  Artificial Sequence

<220>
<223>  Synthetic DNA

<400>  96
ttcaggtaat aaaaggcycg                                                    20


<210>  97
<211>  20
<212>  DNA
<213>  Artificial Sequence

<220>
<223>  Synthetic DNA

<400>  97
ttcaggtaat aaaagtgatc                                                    20


<210>  98

<211>  20
<212>  DNA
<213>  Artificial Sequence

<220>
<223>  Synthetic DNA

<400>  98
ttcaggtaat aaaagggagg                                                    20


<210>  99
<211>  20
<212>  DNA
<213>  Artificial Sequence

<220>
<223>  Synthetic DNA

<400>  99
ttcaggtaat aaaaggctgc                                                    20


<210>  100
<211>  20
<212>  DNA
<213>  Artificial Sequence

<220>
<223>  Synthetic DNA

<400>  100
ttcaggtaat aaaaggaacc                                                    20


<210>  101
<211>  20
<212>  DNA
<213>  Artificial Sequence

<220>
<223>  Synthetic DNA

<400>  101
ttcaggtaat aaaaggctga                                                    20


<210>  102
<211>  20
<212>  DNA
<213>  Artificial Sequence

<220>
<223>  Synthetic DNA

<400>  102
ttcaggtaat aaaaggctgg                                                    20


<210>  103
<211>  20
<212>  DNA
<213>  Artificial Sequence

<220>
<223>  Synthetic DNA

<400>  103
ttcaggtaat aaaagggtg                                                    20


<210>  104
<211>  20
<212>  DNA
<213>  Artificial Sequence

<220>
<223>  Synthetic DNA

<400>  104
ttcaggtaat aaaaggtctg                                                   20


<210>  105
<211>  20
<212>  DNA
<213>  Artificial Sequence

<220>
<223>  Synthetic DNA

<400>  105
ttcaggtaat aaaagggaag                                                   20


<210>  106
<211>  20
<212>  DNA
<213>  Artificial Sequence

<220>
<223>  Synthetic DNA

<400>  106
ttcaggtaat aaaaggtcgt                                                   20


<210>  107
<211>  20
<212>  DNA
<213>  Artificial Sequence

<220>
<223>  Synthetic DNA

<400>  107
ttcaggtaat aaaagttata                                                   20


<210>  108
<211>  20
<212>  DNA
<213>  Artificial Sequence

<220>
<223>  Synthetic DNA

<400>  108
ttcaggtaat aaaaggctcg                                                   20


<210>  109
<211>  20
<212>  DNA
<213>  Artificial Sequence

<220>
<223>  Synthetic DNA

<400>  109
ttcaggcgat aaaaggcgtt                                                   20


<210>  110
<211>  20
<212>  DNA
<213>  Artificial Sequence

<220>
<223>  Synthetic DNA

<400>  110
tgttttggta tattccatac                                                   20


<210>  111
<211>  48
<212>  DNA
<213>  Artificial Sequence

<220>
<223>  Synthetic DNA

<400>  111
ttatttaatg atccaatgga gggggtgatt caggtaataa aaggcctt                    48


<210>  112
<211>  48
<212>  DNA
<213>  Artificial Sequence

<220>
<223>  Synthetic DNA

<400>  112
ttatttaatg atccaatgga gggggtgatt caggtaataa aaggcact                    48


<210>  113
<211>  48
<212>  DNA
<213>  Artificial Sequence

<220>
<223>  Synthetic DNA

<400>  113
ttatttaatg atccaatgga gggggtgatt caggtaataa aaggctta                    48

<210>  114
<211>  48
<212>  DNA
<213>  Artificial Sequence

<220>
<223>  Synthetic DNA

<400>  114
ttatttaatg atccaatgga gggggtgatt caggtaataa aaggcttg                    48


<210>  115
<211>  48
<212>  DNA
<213>  Artificial Sequence

<220>
<223>  Synthetic DNA

<400>  115
ttatttaatg atccaatgga gggggtgatt caggtaataa aaggcctc                    48


<210>  116
<211>  48
<212>  DNA
<213>  Artificial Sequence

<220>
<223>  Synthetic DNA

<400>  116
ttatttaatg atccaatgga gggggtgatt caggtaataa aaggcacg                    48


<210>  117
<211>  48
<212>  DNA
<213>  Artificial Sequence

<220>
<223>  Synthetic DNA

<400>  117
ttatttaatg atccaatgga gggggtgatt caggtaataa aagtcatg                    48


<210>  118
<211>  48
<212>  DNA
<213>  Artificial Sequence

<220>
<223>  Synthetic DNA

<400>  118
ttatttaatg atccaatgga gggggtgatt caggtaataa aaggctrt                    48


<210>  119

<211>  48
<212>  DNA
<213>  Artificial Sequence

<220>
<223>  Synthetic DNA

<400>  119
ttatttaatg atccaatgga gggggtgatt caggtaataa aaggcttr                    48


<210>  120
<211>  48
<212>  DNA
<213>  Artificial Sequence

<220>
<223>  Synthetic DNA

<400>  120
ttatttaatg atccaatgga gggggtgatt caggtaataa aaggcagc                    48


<210>  121
<211>  48
<212>  DNA
<213>  Artificial Sequence

<220>
<223>  Synthetic DNA

<400>  121
ttatttaatg atccaatgga gggggtgatt caggtaataa aaggcycg                    48


<210>  122
<211>  48
<212>  DNA
<213>  Artificial Sequence

<220>
<223>  Synthetic DNA

<400>  122
ttatttaatg atccaatgga gggggtgatt caggtaataa aagtgatc                    48


<210>  123
<211>  48
<212>  DNA
<213>  Artificial Sequence

<220>
<223>  Synthetic DNA

<400>  123
ttatttaatg atccaatgga gggggtgatt caggtaataa aagggagg                    48


<210>  124
<211>  48
<212>  DNA
<213>  Artificial Sequence

<220>
<223>  Synthetic DNA

<400>  124
ttatttaatg atccaatgga gggggtgatt caggtaataa aaggctgc                    48


<210>  125
<211>  48
<212>  DNA
<213>  Artificial Sequence

<220>
<223>  Synthetic DNA

<400>  125
ttatttaatg atccaatgga gggggtgatt caggtaataa aaggaacc                    48


<210>  126
<211>  48
<212>  DNA
<213>  Artificial Sequence

<220>
<223>  Synthetic DNA

<400>  126
ttatttaatg atccaatgga gggggtgatt caggtaataa aaggctga                    48


<210>  127
<211>  48
<212>  DNA
<213>  Artificial Sequence

<220>
<223>  Synthetic DNA

<400>  127
ttatttaatg atccaatgga gggggtgatt caggtaataa aaggctgg                    48


<210>  128
<211>  48
<212>  DNA
<213>  Artificial Sequence

<220>
<223>  Synthetic DNA

<400>  128
ttatttaatg atccaatgga gggggtgatt caggtaataa aaggggtg                    48


<210>  129
<211>  48
<212>  DNA
<213>  Artificial Sequence

<220>
<223>  Synthetic DNA

<400>  129
ttatttaatg atccaatgga gggggtgatt caggtaataa aaggtctg                    48


<210>  130
<211>  48
<212>  DNA
<213>  Artificial Sequence

<220>
<223>  Synthetic DNA

<400>  130
ttatttaatg atccaatgga gggggtgatt caggtaataa aagggaag                    48


<210>  131
<211>  48
<212>  DNA
<213>  Artificial Sequence

<220>
<223>  Synthetic DNA

<400>  131
ttatttaatg atccaatgga gggggtgatt caggtaataa aaggtcgt                    48


<210>  132
<211>  48
<212>  DNA
<213>  Artificial Sequence

<220>
<223>  Synthetic DNA

<400>  132
ttatttaatg atccaatgga gggggtgatt caggtaataa aagttata                    48


<210>  133
<211>  48
<212>  DNA
<213>  Artificial Sequence

<220>
<223>  Synthetic DNA

<400>  133
ttatttaatg atccaatgga gggggtgatt caggtaataa aaggctcg                    48


<210>  134
<211>  48
<212>  DNA
<213>  Artificial Sequence

<220>
<223>  Synthetic DNA

<400>  134
ttatttaatg atccaatgga gggggtgatt caggcgataa aaggcgtt                    48

<210>  135
<211>  21
<212>  DNA
<213>  Stevia rebaudiana

<400>  135
aaccgaatac cgatccaaac c                                                21


<210>  136
<211>  21
<212>  DNA
<213>  Stevia rebaudiana

<400>  136
ttgtatcttg aagttaatca a                                                21


<210>  137
<211>  21
<212>  DNA
<213>  Stevia rebaudiana

<400>  137
acccgtttaa gtgtaaatct t                                                21


<210>  138
<211>  38
<212>  DNA
<213>  Stevia rebaudiana

<400>  138
attaaaattt tataacaata tcatacttga attaaaga                              38


<210>  139
<211>  41
<212>  DNA
<213>  Stevia rebaudiana

<400>  139
ctcgccaaac aaccgaatac cgatccaaac cctgaaatga g                          41


<210>  140
<211>  41
<212>  DNA
<213>  Stevia rebaudiana

<400>  140
tgttaattta ttgtatcttg aagttaatca agagatgctc t                          41


<210>  141
<211>  41
<212>  DNA
<213>  Stevia rebaudiana

<400>  141
agaagacgaa acccgtttaa gtgtaaatct tgaaaacaca t                          41

<210>  142
<211>  58
<212>  DNA
<213>  Stevia rebaudiana

<400>  142
ttatttctaa attaaaattt tataacaata tcatacttga attaaagata acataata        58


<210>  143
<211>  61
<212>  DNA
<213>  Stevia rebaudiana

<400>  143
aaaccctaat ctcgccaaac aaccgaatac cgatccaaac cctgaaatga gcacaactct        60

t                                                                        61


<210>  144
<211>  61
<212>  DNA
<213>  Stevia rebaudiana

<400>  144
tttaaactta tgttaattta ttgtatcttg aagttaatca agagatgctc tcttggagaa        60

a                                                                        61


<210>  145
<211>  61
<212>  DNA
<213>  Stevia rebaudiana

<400>  145
tgaaaaccct agaagacgaa acccgtttaa gtgtaaatct tgaaaacaca ttctttgatg        60

a                                                                        61


<210>  146
<211>  78
<212>  DNA
<213>  Stevia rebaudiana

<400>  146
aacatatttt ttatttctaa attaaaattt tataacaata tcatacttga attaaagata        60

acataatatt tattttta                                                      78


<210>  147
<211>  21
<212>  DNA
<213>  Stevia rebaudiana

<400>  147
aaaaggcatt cgtatggaat a                                                  21

<210>  148
<211>  41
<212>  DNA
<213>  Stevia rebaudiana

<400>  148
ttcaggtaat aaaaggcatt cgtatggaat ataccaaaac a                          41


<210>  149
<211>  61
<212>  DNA
<213>  Stevia rebaudiana

<400>  149
gagggggtga ttcaggtaat aaaaggcatt cgtatggaat ataccaaaac attgcgattc      60

g                                                                      61


<210>  150
<211>  400
<212>  DNA
<213>  Stevia rebaudiana

<400>  150
ctaaccacta tgtttgcagg atattgatag atggaggagg ctccgtcaac atcatccagt      60

ttgaagcact caagaggatg aatatcccgg aatcagagat aaccaccaaa tcggttatcc     120

tgattgggtt cagtggagaa gccaaagcta cctatgggga gatcaagctt ccggtgtata     180

tagaagggat caacatgatt tagaaattct gtgtcatgga ctccctatct agttacaacg     240

tgatccttgg tcgcccatgg atccatgata tgaaggcagt tccatcaacg taccaccagt     300

gcgttaagct tcccactcct tggggagttg taaggatcaa aagtgatcag caagaagcca     360

aagattgcta caccgcgtca atgaaggcta caaaagaagc                           400


<210>  151
<211>  15
<212>  DNA
<213>  Artificial Sequence

<220>
<223>  Synthetic DNA

<400>  151
gtcaacatca tccag                                                       15


<210>  152
<211>  15
<212>  DNA
<213>  Artificial Sequence

<220>
<223>  Synthetic DNA

<400>  152

gaactgcctt catat                                                    15


<210>  153
<211>  21
<212>  DNA
<213>  Stevia rebaudiana

<400>  153
caacatgatt cagaaattct g                                             21


<210>  154
<211>  41
<212>  DNA
<213>  Stevia rebaudiana

<400>  154
tagaagggat caacatgatt cagaaattct gtgtcatgga c                       41


<210>  155
<211>  61
<212>  DNA
<213>  Stevia rebaudiana

<400>  155
ccggtgtata tagaagggat caacatgatt cagaaattct gtgtcatgga ctccctatct   60

a                                                                   61


<210>  156
<211>  25
<212>  DNA
<213>  Artificial Sequence

<220>
<223>  Synthetic DNA

<400>  156
gtcaacatca tccagtttga agcac                                         25


<210>  157
<211>  25
<212>  DNA
<213>  Artificial Sequence

<220>
<223>  Synthetic DNA

<400>  157
gaactgcctt catatcatgg atcca                                         25


<210>  158
<211>  238
<212>  DNA
<213>  Artificial Sequence

<220>
<223>  Synthetic DNA

<400>  158
gtcaacatca tccagtttga agcactcaag aggatgaata tcccggaatc agagataacc      60

accaaatcgg ttatcctgat tgggttcagt ggagaagcca aagctaccta tggggagatc     120

aagcttccgg tgtatataga agggatcaac atgattcaga aattctgtgt catggactcc     180

ctatctagtt acaacgtgat ccttggtcgc ccatggatcc atgatatgaa ggcagttc       238


<210>  159
<211>  238
<212>  DNA
<213>  Artificial Sequence

<220>
<223>  Synthetic DNA

<400>  159
gtcaacatca tccagtttga agcactcaag aggatgaata tcccggaatc agagataacc      60

accaaatcgg ttatcctgat tgggttcagt ggagaagcca aagctaccta tggggagatc     120

aagcttccgg tgtatataga agggatcaac atgatttaga aattctgtgt catggactcc     180

ctatctagtt acaacgtgat ccttggtcgc ccatggatcc atgatatgaa ggcagttc       238


<210>  160
<211>  51
<212>  DNA
<213>  Artificial Sequence

<220>
<223>  Synthetic DNA

<400>  160
gtcaacatca tccagtttga agcactcaag aggatgaata tcccggaatc a               51


<210>  161
<211>  107
<212>  DNA
<213>  Artificial Sequence

<220>
<223>  Synthetic DNA

<400>  161
gagataacca ccaaatcggt tatcctgatt gggttcagtg gagaagccaa agctacctat      60

ggggagatca agcttccggt gtatatagaa gggatcaaca tgattca                   107


<210>  162
<211>  80
<212>  DNA
<213>  Artificial Sequence

<220>
<223>  Synthetic DNA

<400>  162
gaaattctgt gtcatggact ccctatctag ttacaacgtg atccttggtc gcccatggat      60

ccatgatatg aaggcagttc                                                  80


<210>  163
<211>  187
<212>  DNA
<213>  Artificial Sequence

<220>
<223>  Synthetic DNA

<400>  163
gagataacca ccaaatcggt tatcctgatt gggttcagtg gagaagccaa agctacctat      60

ggggagatca agcttccggt gtatatagaa gggatcaaca tgatttagaa attctgtgtc     120

atggactccc tatctagtta caacgtgatc cttggtcgcc catggatcca tgatatgaag     180

gcagttc                                                               187


<210>  164
<211>  400
<212>  DNA
<213>  Stevia rebaudiana

<400>  164
ctaaccacta tgtttgcagg atattgatag atggaggagg ctccgtcaac atcatccagt      60

ttgaagcact caagaggatg aatatcccgg aatcagagat aaccaccaaa tcggttatcc     120

tgattgggtt cagtggagaa gccaaagcta cctatgggga gatcaagctt ccggtgtata     180

tagaagggat caacatgatt cagaaattct gtgtcatgga ctccctatct agttacaacg     240

tgatccttgg tcgcccatgg atccatgata tgaaggcagt tccatcaacg taccaccagt     300

gcgttaagct tcccactcct tggggagttg taaggatcaa aagtgatcag caagaagcca     360

aagattgcta caccgcgtca atgaaggcta caaaagaagc                           400


<210>  165
<211>  21
<212>  DNA
<213>  Stevia rebaudiana

<400>  165
caacatgatt tagaaattct g                                                21


<210>  166
<211>  41
<212>  DNA
<213>  Stevia rebaudiana

<400>  166
tagaagggat caacatgatt tagaaattct gtgtcatgga c                          41

<210>  167
<211>  61
<212>  DNA
<213>  Stevia rebaudiana

<400>  167
ccggtgtata tagaagggat caacatgatt tagaaattct gtgtcatgga ctccctatct        60

a                                                                        61

## Description

### TECHNICAL FIELD

The present invention relates to a stevia plant with low content of tyramine.

### BACKGROUND ART

Tyramine is a biogenic amine that is often detected in foods such as dairy products or fish products. Tyramine has a vasoconstrictive effect and may cause migraine triggered by foods, increased outputs, nausea, vomiting, respiratory disorder, elevated blood glucose levels, etc. (Non-Patent Literature 1). Although tyramine is metabolized and inactivated by monoamine oxidase, the metabolism of tyramine is suppressed in subjects receiving a monoamine oxidase inhibitor so that the subjects are predisposed to develop the symptoms as described above.

### CITATION LIST

### Non-Patent Literature

Non-patent Literature 1: Gardini et al., Front Microbiol. 2016;7:1218

### DISCLOSURE OF THE INVENTION

### Problems to be Solved by the Invention

In response to recently growing health consciousness, there have been increasing needs for highly safe foods.

### Means for Solving the Problems

The present invention provides a stevia plant with low tyramine content, a method of producing the plant, and a method of screening for the plant.

In one aspect, the present invention provides the following.
[1] A low tyramine-content stevia plant having a tyramine content of less than 0.092% per unit mass of dried leaf.
[2] The plant according to [1], having a rebaudioside D content of 1% or more per unit mass of dried leaf.
[3] The plant according to [1] or [2], wherein the plant is homozygous for the allele wherein the base at the position corresponding to position 201 of SEQ ID NO: 150 is C.
[4] The plant according to any one of [1] to [3], wherein the plant is homozygous for the allele wherein the base at the position corresponding to position 201 of SEQ ID NO: 1 is A.
[5] The plant according to any one of [1] to [4], further having at least one of the following genetic features.
   (1) Homozygous for the allele wherein the base at the position corresponding to position 40 of SEQ ID NO: 2 is T.
   (2) Homozygous for the allele wherein the base at the position corresponding to position 44 of SEQ ID NO: 3 is T.
   (3) Homozygous for the allele wherein the base at the position corresponding to position 41 of SEQ ID NO: 4 is C.
   (4) Homozygous for the allele wherein the portion corresponding to positions 55-72 of SEQ ID NO: 5 is deleted.
[6] The plant according to any one of [1] to [5], wherein the plant is heterozygous for the allele wherein the base at the position corresponding to position 49 of SEQ ID NO: 6 is A.
[7] The plant according to any one of [1] to [6], wherein the plant is a non-genetically modified plant.
[8] The plant according to any one of [1] to [7], wherein the plants include stevia plants subjected to mutagenesis treatment and progeny plants thereof.
[9] A seed, tissue, tissue culture or cell of a plant according to any one of [1] to [8].
[10] The tissue, tissue culture or cell according to [9], which is selected from an embryo, a meristem cell, pollen, leaf, root, root apex, petal, protoplast, leaf section and callus.
[11] A method of producing a low tyramine-content stevia plant having a tyramine content of less than 0.092% per unit mass of dried leaf, the method comprising a step of crossing a plant according to any one of [1] to [8] with a second stevia plant.
[12] The method according to [11], wherein the second plant is a plant according to any one of [1] to [8].
[13] An extract of a plant according to any one of [1] to [8], or of a seed, tissue, tissue culture or cell according to [9] or [10], wherein the extract has a low tyramine content.
[14] A method of producing a low tyramine-content stevia extract, comprising a step of obtaining an extract from a plant according to any one of [1] to [8], or from a seed, tissue, tissue culture or cell according to [9] or 10].
[15] A method of producing a low tyramine-content steviol glycoside purified product, comprising a step of purifying steviol glycosides from a stevia extract according to [13].
[16] The method according to [15], wherein the steviol glycosides include rebaudioside A, rebaudioside B, rebaudioside C, rebaudioside D, rebaudioside E, rebaudioside F, rebaudioside M, rebaudioside N, rebaudioside O, stevioside, steviolbioside, rubusoside, dulcoside A or a combination thereof.
[17] A low tyramine-content steviol glycoside purified product produced by a method according to [15] or [16].
[18] A low tyramine-content food or beverage, sweetener composition, flavor or medicament comprising an extract of a tissue, tissue culture or cell according to [13], or a purified product according to [17].
[19] A method of producing a food or beverage, a sweetener composition, a flavor or a medicament, comprising:
   a step of providing an extract of a tissue, tissue culture or cell according to [13], or a purified product according to [17]; and
   a step of adding the extract or the purified product to a raw material for the food or beverage, sweetener composition, flavor or medicament.
[20] A method of screening for a low tyramine-content stevia plant, comprising a step of detecting from the genome of a test stevia plant the presence and/or the absence of a genetic feature of being homozygous for the allele wherein the base at the position corresponding to position 201 of SEQ ID NO: 150 is C.
[21] The method according to [20], further comprising a step of detecting from the genome of a test stevia plant the presence and/or the absence of a genetic feature of being homozygous for the allele wherein the base at the position corresponding to position 201 of SEQ ID NO: 1 is A.
[22] The method according to [20] or [21], further comprising a step of detecting from the genome of a test stevia plant the presence and/or the absence of the following genetic features (1) to (4).
   (1) Homozygous for the allele wherein the base at the position corresponding to position 40 of SEQ ID NO: 2 is T.
   (2) Homozygous for the allele wherein the base at the position corresponding to position 44 of SEQ ID NO: 3 is T.
   (3) Homozygous for the allele wherein the base at the position corresponding to position 41 of SEQ ID NO: 4 is C.
   (4) Homozygous for the allele wherein the portion corresponding to positions 55-72 of SEQ ID NO: 5 is deleted.
[23] The method according to any one of [20] to [22], further comprising a step of detecting from the genome of a test stevia plant the presence and/or the absence of a genetic feature of being heterozygous for the allele wherein the base at the position corresponding to position 49 of SEQ ID NO: 6 is A.
[24] The method according to any one of [20] to [23], wherein the step of detecting a genetic feature is performed by use of CAPS method, dCAPS method or TaqMan PCR method.
[25] The method according to any one of [20] to [24], further comprising a step of measuring the content of tyramine in a test stevia plant tissue.
[26] A screening kit for a low tyramine-content stevia plant, comprising a reagent for detecting the presence and/or the absence of a genetic feature of being homozygous for the allele wherein the base at the position corresponding to position 201 of SEQ ID NO: 150 is C.
[27] The kit according to [26], further comprising a reagent for detecting the presence and/or the absence of a genetic feature of being homozygous for the allele wherein the base at the position corresponding to position 201 of SEQ ID NO: 1 is A.
[28] The kit according to [26] or [27], further comprising a reagent for detecting the presence and/or the absence of the following genetic features (1) to (4).
   (1) Homozygous for the allele wherein the base at the position corresponding to position 40 of SEQ ID NO: 2 is T.
   (2) Homozygous for the allele wherein the base at the position corresponding to position 44 of SEQ ID NO: 3 is T.
   (3) Homozygous for the allele wherein the base at the position corresponding to position 41 of SEQ ID NO: 4 is C.
   (4) Homozygous for the allele wherein the portion corresponding to positions 55-72 of SEQ ID NO: 5 is deleted.
[29] The kit according to any one of [26] to [28], further comprising a reagent for detecting the presence and/or the absence of a genetic feature of being heterozygous for the allele wherein the base at the position corresponding to position 49 of SEQ ID NO: 6 is A.
[30] The kit according to any one of [26] to [29], wherein the reagent comprises a primer and/or a probe for use in CAPS method, dCAPS method or TaqMan PCR method.
[31] A method of producing a low tyramine-content stevia plant, comprising a step of introducing a variation from T to C to a position corresponding to position 201 of SEQ ID NO: 150.
[32] The method according to [31], wherein the introduction of the variation is performed by a mutagenesis treatment.

### ADVANTAGEOUS EFFECTS OF THE INVENTION

The present invention enables the obtainment of a stevia plant with low tyramine content and the provision of an approach for producing such a plant, a leaf obtainable from such a plant, and a food, drink, etc. containing an extract obtained from this leaf.

### BRIEF DESCRIPTION OF THE DRAWINGS

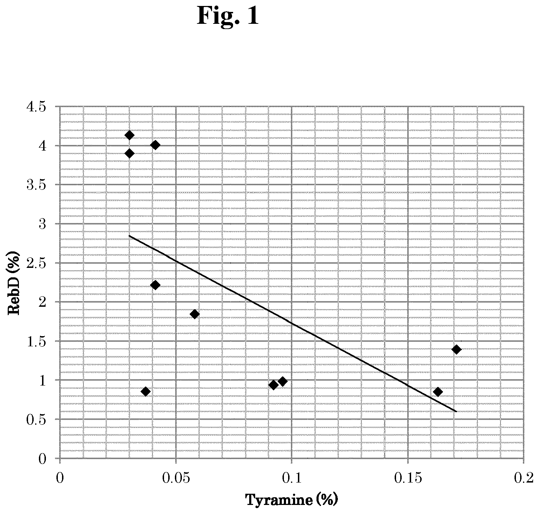

Figure 1 is a diagram showing results of analyzing a correlation of tyramine content with RebD content.

### DESCRIPTION OF EMBODIMENTS

Hereinafter, the present invention will be described in detail. The embodiments are given below merely for illustrating the present invention and are not intended to limit the present invention by such embodiments. The present invention can be carried out in various modes without departing from the spirit of the present invention.

Note that all documents, as well as laid-open application publications, patent application publications, and other patent documents cited herein shall be incorporated herein by reference. The present specification incorporates the contents of the specification and the drawings of Japanese Patent Application No. 2018-248657, filed on December 28, 2018, from which the present application claims priority.

### 1. Low tyramine-content stevia plant

The present invention provides a low tyramine-content stevia plant having a tyramine content of less than 0.092*%* per unit mass of dried leaf (hereinafter, generically referred to as "plant of the present invention" or "stevia plant of the present invention").

Stevia is a perennial plant of the family *Asteraceae* with Paraguay in the South America as its place of origin, and its scientific name is *Stevia rebaudiana* Bertoni.

The feature "having a tyramine content of less than 0.092% per unit mass of dried leaf" means that, for example, tyramine is contained at a ratio of less than 0.092% by mass (e.g. less than 0.046 mg) in dried leaf having a predetermined mass (e.g. 50 mg). The tyramine content in a plant of the present invention is not limited and may be, for example, 0.090% or less, 0.080% or less, 0.070% or less, 0.060% or less, 0.058% or less, 0.057% or less, 0.056% or less, 0.055% or less, 0.054% or less, 0.053% or less, 0.052% or less, 0.051% or less, 0.050% or less, 0.049% or less, 0.048% or less, 0.047% or less, 0.046% or less, 0.045% or less, 0.044% or less, 0.043% or less, 0.042% or less, 0.041% or less, 0.040% or less, 0.039% or less, 0.038% or less, 0.037% or less, 0.036% or less, 0.035% or less, 0.034% or less, 0.033% or less, 0.032% or less, 0.031*%* or less, 0.030% or less, 0.029% or less, 0.028% or less, 0.027% or less, 0.026% or less, 0.025% or less, 0.024% or less, 0.023% or less, 0.022% or less, 0.021% or less, 0.020% or less, 0.019% or less, 0.018% or less, 0.017% or less, 0.016% or less, 0.015% or less, 0.014% or less, 0.013% or less, 0.012% or less, 0.011% or less, or 0.010% or less per unit mass of dried leaf. A lower tyramine content in the plant of the present invention is more preferred and is more preferably 0.058% or less, particularly preferably 0.030% or less.

In this context, dried leaf refers to leaf having a water content decreased to 3 to 4% by weight by drying fresh leaves of the stevia plant of the present invention.

The plant of the present invention may contain 1% or more of RebD per unit mass of dried leaf. In this embodiment, the ratio of RebD per unit mass of dried leaf is not limited and may be, for example, 1*%* or more, 1.5% or more, 2% or more, 2.5% or more, 3% or more, 3.3% or more, 3.4% or more, 3.5% or more, 3.6% or more, 3.7% or more, 3.8% or more, 3.9% or more, 4.0% or more, 4.1% or more, 4.2% or more, 4.3% or more, 4.4% or more, 4.5% or more, 4.6% or more, 4.7% or more, 4.8% or more, 4.9% or more, 5.0% or more, 5.1% or more, 5.2% or more, 5.3% or more, 5.4% or more, 5.5% or more, 5.6% or more, 5.7% or more, 5.8% or more, 5.9% or more, 6.0% or more, or the like, and is preferably 3.3% or more, more preferably 3.6% or more. The upper limit of the ratio of RebD per unit mass of dried leaf is not particularly limited and may be, for example, 20%, 15% or 10%.

In one embodiment, the stevia plant of the present invention has a genetic feature of being homozygous for the allele wherein the base at the position corresponding to position 201 of SEQ ID NO: 150 is C (hereinafter, referred to as the "genetic feature X of the present invention").

In one embodiment, the stevia plant of the present invention has a genetic feature of being homozygous for the allele wherein the base at the position corresponding to position 201 of SEQ ID NO: 1 is A (hereinafter, referred to as the "genetic feature A of the present invention").

In another embodiment, the stevia plant of the present invention has at least one of the following genetic features (B-1) to (B-4) (hereinafter, referred to as the "genetic feature B of the present invention").
(B-1) Homozygous for the allele wherein the base at the position corresponding to position 40 of SEQ ID NO: 2 is T (hereinafter, referred to as the "genetic feature B-1 of the present invention").
(B-2) Homozygous for the allele wherein the base at the position corresponding to position 44 of SEQ ID NO: 3 is T (hereinafter, referred to as the "genetic feature B-2 of the present invention").
(B-3) Homozygous for the allele wherein the base at the position corresponding to position 41 of SEQ ID NO: 4 is C (hereinafter, referred to as the "genetic feature B-3 of the present invention").
(B-4) Homozygous for the allele wherein the portion corresponding to positions 55-72 of SEQ ID NO: 5 is deleted (hereinafter, referred to as the "genetic feature B-4 of the present invention").

In an alternative embodiment, the stevia plant of the present invention has a genetic feature of being heterozygous for the allele wherein the base at the position corresponding to position 49 of SEQ ID NO: 6 is A (hereinafter, referred to as the "genetic feature C of the present invention").

In a preferable embodiment, the stevia plant of the present invention has the genetic feature X and the genetic feature A of the present invention. In another preferable embodiment, the stevia plant of the present invention has the genetic feature X and the genetic feature B (i.e. at least one of the genetic features B-1 to B-4 of the present invention) of the present invention. In an alternative preferable embodiment, the stevia plant of the present invention has the genetic feature X and the genetic feature C of the present invention. In an alternative preferable embodiment, the stevia plant of the present invention has the genetic feature X, the genetic feature A, and the genetic feature B (i.e. at least one of the genetic features B-1 to B-4 of the present invention) of the present invention. In an alternative preferable embodiment, the stevia plant of the present invention has, the genetic feature X, the genetic feature A, and the genetic feature C of the present invention. In an alternative preferable embodiment, the stevia plant of the present invention has the genetic feature X, the genetic feature B, and the genetic feature C of the present invention. In a more preferable embodiment, the stevia plant of the present invention has all of the genetic features X, A, B and C of the present invention.

The phrase "position (or portion) corresponding to" means the following. In case a sequence identical to a reference sequence (e.g. SEQ ID NOs: 1 to 6, 150 etc.) is present in the genome, it means a position or a portion in the sequence (e.g. 201, 40, 44, 41, 55-72, 49, etc.) present in the genome, and in case a sequence identical to the reference sequence is not present in the genome, it means a position or portion in a sequence in the genome corresponding to the reference sequence, which corresponds to the position or portion in the reference sequence. Whether or not a sequence identical to or corresponding to the reference sequence exists in the genome can be determined by, for example, amplifying genomic DNA of the stevia plant of interest with a primer capable of amplifying the reference sequence by PCR, sequencing the amplified product, and performing alignment analysis between the obtained sequence and the reference sequence. Non-limiting examples of a sequence corresponding to a reference sequence include, for example, a nucleotide sequence having a sequence identity of 60% or more, 70% or more, 75% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, 98.1*%* or more, 98.4% or more, 98.7% or more, 99% or more, 99.2% or more, 99.5 or more, or 99.8% or more to the reference sequence. The position or portion corresponding to the position or portion in the reference sequence in the sequence corresponding to the reference sequence in the genome can be determined by taking into account the nucleotide sequence before and after the position or portion in the reference sequence and the like. For example, a position or portion in the sequence corresponding to the reference sequence in the genome corresponding to a position or portion in the reference sequence can be determined by an alignment analysis of a reference sequence with a sequence corresponding to a reference sequence in the genome.

For instance, when taking "the position corresponding to position 201 of SEQ ID NO: 1" of the genetic feature A of the present invention as an example, in case the genome of a stevia plant has a portion consisting of a nucleotide sequence identical to SEQ ID NO: 1, "the position corresponding to position 201 of SEQ ID NO: 1" is position 201 from the 5' end of the portion consisting of a nucleotide sequence identical to SEQ ID NO: 1 in the genome. On the other hand, in case the genome of a stevia plant has a portion consisting of a nucleotide sequence which is not identical to, but which corresponds to SEQ ID NO: 1, the genome does not have a portion consisting of a nucleotide sequence identical to SEQ ID NO: 1. Therefore, "the position corresponding to position 201 of SEQ ID NO: 1" does not necessarily correspond to position 201 from the 5' end of the portion corresponding to SEQ ID NO: 1. However, it is possible to identify "the position corresponding to position 201 of SEQ ID NO: 1" in the genome of such a stevia plant by taking into account the nucleotide sequence before and after the position 201 of SEQ ID NO: 1, and the like. For instance, one can identify "the position corresponding to position 201 of SEQ ID NO: 1" in the genome of a stevia plant by an alignment analysis of the nucleotide sequence of a portion corresponding to SEQ ID NO: 1" in the genome of a stevia plant and the nucleotide sequence of SEQ ID NO: 1.

"The portion consisting of a nucleotide sequence corresponding to SEQ ID NO: 1" means, for instance, a portion consisting of a nucleotide sequence having a sequence identity of 60% or more, 70% or more, 75% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, 98.1*%* or more, 98.4% or more, 98.7% or more, 99% or more, 99.2% or more, 99.5% or more, or 99.8% or more to the nucleotide sequence of SEQ ID NO: 1.

In one embodiment, "the portion consisting of a nucleotide sequence corresponding to SEQ ID NO: 1" includes a portion of the genome of a stevia plant which can be amplified by PCR using a forward primer which hybridizes to a complementary sequence of a portion of 15 to 25 base long from the 5' end of SEQ ID NO: 1 and a reverse primer which hybridizes to a portion of 15 to 25 base long from the 3' end of SEQ ID NO: 1.

For simplicity, the genetic feature A of the present invention is used here as an example for explanation, but the same applies to the genetic features X, B (including the genetic features B-1 to B-4) and C of the present invention.

In a specific embodiment, "the portion consisting of a nucleotide sequence corresponding to SEQ ID NO: 150" includes, for instance, a portion of the genome of a stevia plant which can be amplified by PCR using a forward primer comprising the nucleotide sequence of SEQ ID NO: 151 and a reverse primer comprising the nucleotide sequence of SEQ ID NO: 152.

In a specific embodiment, "the portion consisting of a nucleotide sequence corresponding to SEQ ID NO: 1" includes, for instance, a portion of the genome of a stevia plant which can be amplified by PCR using a forward primer comprising the nucleotide sequence of SEQ ID NO: 7 and a reverse primer comprising the nucleotide sequence of SEQ ID NO: 8.

In a specific embodiment, "the portion consisting of a nucleotide sequence corresponding to SEQ ID NO: 2" includes, for instance, a portion of the genome of a stevia plant which can be amplified by PCR using a forward primer comprising the nucleotide sequence of SEQ ID NO: 9 and a reverse primer comprising the nucleotide sequence of SEQ ID NO: 10.

In a specific embodiment, "the portion consisting of a nucleotide sequence corresponding to SEQ ID NO: 3" includes, for instance, a portion of the genome of a stevia plant which can be amplified by PCR using a forward primer comprising the nucleotide sequence of SEQ ID NO: 11 and a reverse primer comprising the nucleotide sequence of SEQ ID NO: 12.

In a specific embodiment, "the portion consisting of a nucleotide sequence corresponding to SEQ ID NO: 4" includes, for instance, a portion of the genome of a stevia plant which can be amplified by PCR using a forward primer comprising the nucleotide sequence of SEQ ID NO: 13 and a reverse primer comprising the nucleotide sequence of SEQ ID NO: 14.

In a specific embodiment, "the portion consisting of a nucleotide sequence corresponding to SEQ ID NO: 5" includes, for instance, a portion of the genome of a stevia plant which can be amplified by PCR using a forward primer comprising the nucleotide sequence of SEQ ID NO: 15 and a reverse primer comprising the nucleotide sequence of SEQ ID NO: 16.

In a specific embodiment, "the portion consisting of a nucleotide sequence corresponding to SEQ ID NO: 6" includes, for instance, a portion of the genome of a stevia plant which can be amplified by PCR using a forward primer comprising the nucleotide sequence of SEQ ID NO: 17 and a reverse primer comprising the nucleotide sequence of SEQ ID NO: 18.

In a specific embodiment, "the allele wherein the base at the position corresponding to position 201 of SEQ ID NO: 150 is C" comprises the nucleotide sequence of SEQ ID NO: 153, 154 or 155.

In a specific embodiment, "the allele wherein the base at the position corresponding to position 201 of SEQ ID NO: 1 is A" comprises the nucleotide sequence of SEQ ID NO: 19, 20 or 21.

In a specific embodiment, "the allele wherein the base at the position corresponding to position 40 of SEQ ID NO: 2 is T" comprises the nucleotide sequence of SEQ ID NO: 22, 23 or 24.

In a specific embodiment, "the allele wherein the base at the position corresponding to position 44 of SEQ ID NO: 3 is T" comprises the nucleotide sequence of SEQ ID NO: 25, 26 or 27.

In a specific embodiment, "the allele wherein the base at the position corresponding to position 41 of SEQ ID NO: 4 is C" comprises the nucleotide sequence of SEQ ID NO: 28, 29 or 30.

In a specific embodiment, "the allele wherein the portion corresponding to positions 55-72 of SEQ ID NO: 5 is deleted" comprises the nucleotide sequence of SEQ ID NO: 31, 32 or 33.

In a specific embodiment, "the allele wherein the base at the position corresponding to position 49 of SEQ ID NO: 6 is A" comprises the nucleotide sequence of SEQ ID NO: 34, 35 or 36.

Here, a position selected from the group consisting of (X) a position corresponding to position 201 of SEQ ID NO: 150, (A) a position corresponding to position 201 of SEQ ID NO: 1, (B-1) a position corresponding to position 40 of SEQ ID NO: 2, (B-2) a position corresponding to position 44 of SEQ ID NO: 3, (B-3) a position corresponding to position 41 of SEQ ID NO: 4, (B-4) a portion corresponding to positions 55-72 of SEQ ID NO: 5, and (C) a position corresponding to position 49 of SEQ ID NO: 6 may be generically referred to as a "polymorphic site of the present invention" or a "variation site of the present invention".

Also, a variation selected from the group consisting of (X) a variation from T to C at a position corresponding to position 201 of SEQ ID NO: 150, (A) a variation from C to A at a position corresponding to position 201 of SEQ ID NO: 1, (B-1) a variation from A to T at a position corresponding to position 40 of SEQ ID NO: 2, (B-2) a variation from C to T at a position corresponding to position 44 of SEQ ID NO: 3, (B-3) a variation from G to C at a position corresponding to position 41 of SEQ ID NO: 4, (B-4) a deletion of the portion corresponding to positions 55-72 of SEQ ID NO: 5, and (C) a variation from C to A at a position corresponding to position 49 of SEQ ID NO: 6 may be generically referred to as a "polymorphism of the present invention" or a "variation of the present invention".

The above genetic features can be detected by PCR method, TaqMan PCR method, sequencing method, microarray method, Invader method, TILLING method, RAD (random amplified polymorphic DNA) method, restriction fragment length polymorphism (RFLP) method, PCR-SSCP method, AFLP (amplified fragment length polymorphism) method, SSLP (simple sequence length polymorphism) method, CAPS (cleaved amplified polymorphic sequence) method, dCAPS (derived cleaved amplified polymorphic sequence) method, allele-specific oligonucleotide (ASO) method, ARMS method, denaturing gradient gel electrophoresis (DGGE) method, CCM (chemical cleavage of mismatch) method, DOL method, MALDI-TOF/MS method, TDI method, padlock probe method, molecular beacon method, DASH (dynamic allele specific hybridization) method, UCAN method, ECA method, PINPOINT method, PROBE (primer oligo base extension) method, VSET (very short extension) method, Survivor assay, Sniper assay, Luminex assay, GOOD method, LCx method, SNaPshot method, Mass ARRAY method, pyrosequencing method, SNP-IT method, melting curve analysis method, etc., but detection methods are not limited thereto.

In a specific embodiment, each genetic feature of the present invention is detectable using the following combination of a primer set and a restriction enzyme.

In case a candidate plant has the genetic feature X, for example, bands of approximately 51 bp long (e.g. SEQ ID NO: 160), approximately 107 bp (e.g. SEQ ID NO: 161) and approximately 80 bp (e.g. SEQ ID NO: 162) are obtained by: performing PCR amplification using a forward primer having the nucleotide sequence shown in SEQ ID NO: 156 and a reverse primer having the nucleotide sequence shown in SEQ ID NO: 157 on the genomic DNA of the candidate plant; and treating the obtained PCR product (approximately 238 bp long, e.g. SEQ ID NO: 158 or 159) with a restriction enzyme Hpy188I. On the other hand, when restriction enzyme-treated products of approximately 51 bp long (e.g. SEQ ID NO: 160) and approximately 187 bp long (e.g. SEQ ID NO: 163) are formed, the candidate plant does not have the genetic feature X.

In case a candidate plant has the genetic feature A, for example, a band of approximately 96 bp long (e.g. SEQ ID NO: 41) and a band of approximately 100 bp (e.g. SEQ ID NO: 42) are obtained by: performing PCR amplification using a forward primer having the nucleotide sequence shown in SEQ ID NO: 37 and a reverse primer having the nucleotide sequence shown in SEQ ID NO: 38 on the genomic DNA of the candidate plant; and treating the obtained PCR product (approximately 196 bp long, e.g. SEQ ID NO: 39 or 40) with a restriction enzyme Hpy188I. On the other hand, when restriction enzyme-treated products of approximately 43 bp (e.g. SEQ ID NO: 43) and approximately 57 bp (e.g. SEQ ID NO: 44) are formed, the candidate plant does not have the genetic feature A.

In case where a candidate plant has the genetic feature B-1, for example, only a band of approximately 297 bp long (e.g. SEQ ID NO: 47) is obtained by: performing PCR amplification using a forward primer having the nucleotide sequence shown in SEQ ID NO: 45 and a reverse primer having the nucleotide sequence shown in SEQ ID NO: 46 on the genomic DNA of the candidate plant; and treating the obtained PCR product (approximately 297 bp long: e.g. SEQ ID NO: 47 or 48) with a Kpnl restriction enzyme. On the other hand, when a restriction enzyme-treated product of approximately 258 bp (e.g. SEQ ID NO: 49) is formed, the candidate plant does not have the genetic feature B-1.

In case where a candidate plant has the genetic feature B-2, for example, only a band of approximately 383 bp long (e.g. SEQ ID NO: 52) is obtained by: performing PCR amplification using a forward primer having the nucleotide sequence shown in SEQ ID NO: 50 and a reverse primer having the nucleotide sequence shown in SEQ ID NO: 51 on the genomic DNA of the candidate plant; and treating the obtained PCR product (approximately 383 bp long: e.g. SEQ ID NO: 52 or 53) with an Xbal restriction enzyme. On the other hand, when a restriction enzyme-treated product of approximately 344 bp long (e.g. SEQ ID NO: 54) is formed by the Xbal restriction enzyme treatment of the PCR product, the candidate plant does not have the genetic feature B-2.

In case where a candidate plant has the genetic feature B-3, for example, only a band of approximately 390 bp long (e.g. SEQ ID NO: 57) is obtained by: performing PCR amplification using a forward primer having the nucleotide sequence shown in SEQ ID NO: 55 and a reverse primer having the nucleotide sequence shown in SEQ ID NO: 56 on the genomic DNA of the candidate plant; and treating the obtained PCR product (approximately 390 bp long: e.g. SEQ ID NO: 57 or 58) with an AflII restriction enzyme. On the other hand, when a restriction enzyme-treated product of approximately 347 bp long (e.g. SEQ ID NO: 59) is formed, the candidate plant does not have the genetic feature B-3.

In case where a candidate plant has the genetic feature B-4, for example, only a PCR product of approximately 140 bp (e.g. SEQ ID NO: 62) is formed by performing PCR amplification using a forward primer having the nucleotide sequence shown in SEQ ID NO: 60 and a reverse primer having the nucleotide sequence shown in SEQ ID NO: 61 on the genomic DNA of the candidate plant. On the other hand, when PCR products of 140 bp long (e.g. SEQ ID NO: 62) and 158 bp long (e.g. SEQ ID NO: 63) are formed, the candidate plant does not have the genetic feature B-4.

In case a candidate plant has the genetic feature C, for example, a band of approximately 367 bp long (e.g. SEQ ID NO: 66) and a band of approximately 321 bp (e.g. SEQ ID NO: 68) are obtained by: performing PCR amplification using a forward primer having the nucleotide sequence shown in SEQ ID NO: 64 and a reverse primer having the nucleotide sequence shown in SEQ ID NO: 65 on the genomic DNA of the candidate plant; and treating the obtained PCR product (approximately 367 bp long: e.g. SEQ ID NO: 66 or 67) with a restriction enzyme SpeI. On the other hand, when only a restriction enzyme-treated product of approximately 367 bp long (e.g. SEQ ID NO: 67) is formed, the candidate plant does not have the genetic feature C.

The term "approximately" as to bp long described above means ±5 bp. The restriction enzyme treatment can be performed according to conditions recommended by the distributor of each restriction enzyme used.

Tyramine can be extracted and quantified by any known approach or an approach described in Example 1. Non-limiting examples of the method of measuring a tyramine content include the following approaches:
(1) A fresh leaf of the stevia plant is homogenized with an aqueous trichloroacetic acid solution (e.g. a 20% aqueous trichloroacetic acid solution) and left for a predetermined time.
(2) Sodium octanesulfonate (e.g. 0.1 mol/L) is added to the supernatant and mixed, and the mixture is applied to a solid phase extraction device (e.g. Sep-Pak(R) Vac 6cc 1g C18 Cartridges from Waters Corp. are used).
(3) The solid phase extraction device is washed, followed by the elution of amines with an aqueous methanol solution (e.g. an 80% aqueous methanol solution).
(4) The eluate is concentrated (e.g. concentrated 10-fold), if necessary, and an internal standard solution (e.g. a solution of 1,8-diaminooctane dissolved at a concentration of 20 µg/mL in 0.5 mol/L hydrochloric acid), anhydrous sodium carbonate, and a solution of dansyl chloride in acetone are added thereto and well mixed, and the mixture is warmed in a water bath of 45°C for a predetermined time (e.g. 1 hour) with light shielded.
(5) A proline solution (e.g. a 10% proline solution) is added thereto, shaken, and left for a predetermined time (e.g. 10 minutes).
(6) Toluene is added thereto and shaken for a predetermined time (e.g. 1 minute), and the toluene layer is collected as an upper layer. The same operation as above is performed on the remaining solution, if necessary.
(7) The toluene layer is concentrated into dryness under reduced pressure (e.g. at 40°C or lower), and acetonitrile is added to the residue and well mixed.
(8) The obtained solution is analyzed by HPLC.

RebD can be extracted in the state of a liquid extract by reacting fresh leaf or dried leaf of a plant of the present invention with a suitable solvent (an aqueous solvent such as water or an organic solvent such as an alcohol, ether or acetone). For the extraction conditions, etc., see a method described in Ohta et al., J. Appl. Glycosci., Vol. 57, No. 3 (2010) or WO2010/038911, or a method described in Examples mentioned later.

RebD can be further purified from the liquid extract thus obtained by use of a method known in the art such as a gradient of ethyl acetate or any of other organic solvents:water, high performance liquid chromatography (HPLC), gas chromatography, time-of-flight mass spectrometry (TOF-MS), or ultra (high) performance liquid chromatography (UPLC).

The contents of RebD can be measured by a method described in Ohta et al., J. Appl. Glycosci., Vol. 57, No. 3 (2010) or WO2010/038911, or a method described in Examples mentioned later. Specifically, a fresh leaf can be sampled from the stevia plant of the present invention, followed by measurement by LC/MS-MS.

The plant of the present invention may include not only the whole plant but a plant organ (e.g. a leaf, a petal, a stem, a root, and a seed), a plant tissue (e.g. epidermis, phloem, soft tissue, xylem, vascular bundle, palisade tissue, and spongy tissue), various forms of plant cells (e.g. suspended cultured cells), a protoplast, a leaf section, a callus, and the like. The leaf may be the dried leaf mentioned above.

The plant of the present invention may also include a tissue culture or a cultured plant cell. This is because the plant can be regenerated by culturing such a tissue culture or a cultured plant cell. Examples of the tissue culture or the cultured plant cell of the plant of the present invention include, but are not limited to, embryos, meristem cells, pollens, leaves, roots, root apices, petals, protoplasts, leaf sections and calluses.

### 2. Method of producing a plant of the present invention

In an alternative aspect, the present invention provides a method of producing a low tyramine-content stevia plant having a tyramine content of less than 0.092% per unit mass of dried leaf, the method comprising a step of crossing the stevia plant of the present invention with a second stevia plant (hereinafter, may be referred to as the "production method of the present invention").

The "low tyramine-content stevia plant having a tyramine content of less than 0.092% per unit mass of dried leaf" produced by the method has the same phenotype and genetic properties as those of the plant of the present invention.

The ranges of the contents of tyramine and RebD in the plant obtained by the production method of the present invention are as described above about the plant of the present invention.

In one embodiment, the plant obtained by the production method of the present invention has the genetic feature X of the present invention. In one embodiment, the plant obtained by the production method of the present invention has the genetic feature A of the present invention. In another embodiment, the plant obtained by the production method of the present invention has the genetic feature B (i.e. at least one of the genetic features B-1 to B-4 of the present invention) of the present invention. In an alternative embodiment, the plant obtained by the production method of the present invention has the genetic feature C of the present invention. In a preferable embodiment, the plant obtained by the production method of the present invention has the genetic feature X and the genetic feature A of the present invention. In another preferable embodiment, the plant obtained by the production method of the present invention has the genetic feature X and the genetic feature B (i.e. at least one of the genetic features B-1 to B-4 of the present invention) of the present invention. In an alternative preferable embodiment, the plant obtained by the production method of the present invention has the genetic feature X and the genetic feature C of the present invention. In an alternative preferable embodiment, the plant obtained by the production method of the present invention has the genetic feature X, the genetic feature A, and the genetic feature B (i.e. at least one of the genetic features B-1 to B-4 of the present invention) of the present invention. In an alternative preferable embodiment, the plant obtained by the production method of the present invention has the genetic feature X, the genetic feature A, and the genetic feature C of the present invention. In an alternative preferable embodiment, the plant obtained by the production method of the present invention has the genetic feature X, the genetic feature B, and the genetic feature C of the present invention. In a more preferable aspect, the plant obtained by the production method of the present invention has all of the genetic features X, A, B and C of the present invention.

In the production method of the present invention, "hybridizing" means that the plant of the present invention (first generation (S1)) is crossed with a second plant (S1) to obtain a progeny plant thereof (plant produced by the production method of the present invention (second generation (S2)). The hybridizing method is preferably backcross. The "backcross" is an approach of further crossing a progeny plant (S2) generated between the plant of the present invention and the second plant, with the plant of the present invention (i.e. a plant having the genetic feature(s) of the present invention) (S1) to produce a plant having the genetic feature(s) of the present invention. When the second plant (S1) for use in the production method of the present invention has the same phenotype and genetic properties as those of the plant of the present invention, the crossing is substantially backcross. The genetic polymorphism of the present invention is inheritable according to the Mendel's law. In association with this, the phenotype correlating with the genetic polymorphism, i.e. the low tyramine-content phenotype, is also inheritable according to the Mendel's law.

Alternatively, the plant of the present invention can also be produced by selfing. The selfing can be performed by the self-pollination of the stamen pollen of the plant of the present invention with the pistil of the plant of the present invention.

Since the plant produced by the production method of the present invention has the same phenotype and genetic properties as those of the plant of the present invention, the plant produced by the production method of the present invention can be further crossed with a third stevia plant to produce a stevia plant having a phenotype equivalent to that of the plant of the present invention.

In an alternative embodiment, the plant of the present invention may be produced by regenerating a plant by the culture of the tissue culture or the cultured plant cell mentioned above. The culture conditions are the same as those for culturing a tissue culture or a cultured plant cell of the wild type stevia plant and are known in the art (Protocols for in vitro cultures and secondary metabolite analysis of aromatic and medicinal plants, Method in Molecular Biology, vo. 1391, pp. 113-123).

In a further alternative embodiment, the plant of the present invention may be produced by introducing the variation of the present invention to the genome of a stevia plant. The introduction of the variation may be performed by a genetic modification approach or may be performed by a non-genetic modification approach. Examples of the "non-genetic modification approach" include a method of inducing a variation in the gene of a host cell (or a host plant) without transfection with a foreign gene. Examples of such a method include a method of allowing a mutagen to act on a plant cell. Examples of such a mutagen include ethylmethanesulfonic acid (EMS) and sodium azide. For example, the ethylmethanesulfonic acid (EMS) can be used at a concentration such as 0.1%, 0.2%, 0.3%, 0.4%, 0.5%, 0.6%, 0.7%, 0.8%, 0.9%, or 1.0% to treat a plant cell. The treatment time is 1 to 48 hours, 2 to 36 hours, 3 to 30 hours, 4 to 28 hours, 5 to 26 hours, or 6 to 24 hours. The procedures themselves of the treatment are known in the art and can be performed by dipping a water-absorbed seed obtained through a water absorption process in a treatment solution containing the mutagen at the concentration described above for the treatment time described above.

An alternative example of the non-genetic modification approach can be a method of irradiating a plant cell with radiation or light beam such as X ray, y ray, or ultraviolet ray. In this case, a cell irradiated using an appropriate dose (ultraviolet lamp intensity, distance, and time) of ultraviolet ray is cultured in a selective medium or the like, and then, a cell, a callus, or a plant having the trait of interest can be selected. In this operation, the irradiation intensity is 0.01 to 100 Gr, 0.03 to 75 Gr, 0.05 to 50 Gr, 0.07 to 25 Gr, 0.09 to 20 Gr, 0.1 to 15 Gr, 0.1 to 10 Gr, 0.5 to 10 Gr, or 1 to 10 Gr. The irradiation distance is 1 cm to 200 m, 5 cm to 100 m, 7 cm to 75 m, 9 cm to 50 m, 10 cm to 30 m, 10 cm to 20 m, or 10 cm to 10 m. The irradiation time is 1 minute to 2 years, 2 minutes to 1 year, 3 minutes to 0.5 years, 4 minutes to 1 month, 5 minutes to 2 weeks, or 10 minutes to 1 week. The irradiation intensity, distance and time differ depending on the type of radiation or the state of the subject to be irradiated (cell, callus, or plant) and can be appropriately adjusted by those skilled in the art.

Approaches such as cell fusion, anther culture (haploid induction), and remote crossing (haploid induction) are also known in the art.

In general, plant cells may involve a mutation during culture. Therefore, it is preferred to regenerate a plant individual, for more stably maintaining the trait.

The scope of the present invention does not exclude a plant obtained by the ex-post facto genetic recombination (e.g. genome editing) with the plant of the present invention as a host (e.g. a plant further provided with another trait by genetic recombination with the plant of the present invention as a host).

### 3. Method of screening for a plant of the present invention

A plant of the present invention or a plant having the same phenotype and genetic properties as those of the plant of the present invention can be screened for by detecting the genetic feature(s) of the present invention from a tissue of this plant. In this context, "screening" means that the plant of the present invention is discriminated from other plants to select the plant of the present invention.

Thus, in an alternative aspect, the present invention provides a method of screening for a low tyramine-content stevia plant, comprising a step of detecting the presence and/or the absence of at least one of the genetic features X, A, B and C of the present invention from the genome of a test plant (hereinafter, may be referred to as the "screening method of the present invention").

In one embodiment, the genetic feature(s) to be detected is the genetic feature X of the present invention. In another embodiment, the genetic feature(s) to be detected is the genetic feature A of the present invention. In an alternative embodiment, the genetic feature(s) to be detected is the genetic feature B (i.e. at least one of the genetic features B-1 to B-4 of the present invention) of the present invention. In an alternative embodiment, the genetic feature(s) to be detected is the genetic feature C of the present invention. In a preferable embodiment, the genetic feature(s) to be detected is the genetic feature X and the genetic feature A of the present invention. In another preferable embodiment, the genetic feature(s) to be detected is the genetic feature X and the genetic feature B (i.e. at least one of the genetic features B-1 to B-4 of the present invention) of the present invention. In an alternative preferable embodiment, the genetic feature(s) to be detected is the genetic feature X and the genetic feature C of the present invention. In an alternative preferable embodiment, the genetic feature(s) to be detected is the genetic feature X, the genetic feature A, and the genetic feature B (i.e. at least one of the genetic features B-1 to B-4 of the present invention) of the present invention. In an alternative preferable embodiment, the genetic feature(s) to be detected is the genetic feature X, the genetic feature A, and the genetic feature C of the present invention. In an alternative preferable embodiment, the genetic feature(s) to be detected is the genetic feature X, the genetic feature B, and the genetic feature C of the present invention. In a more preferable embodiment, the genetic feature(s) to be detected is all of the genetic features X, A, B and C of the present invention.

The screening method of the present invention may further comprise a step of selecting from among the test plants a plant in which the presence of at least one genetic feature of the above is detected.

The presence of the genetic feature(s) of the present invention can be determined by detecting the presence of an allele selected from the group consisting of:
(X) an allele wherein the base at the position corresponding to position 201 of SEQ ID NO: 150 is C (e.g. an allele comprising the nucleotide sequence of SEQ ID NO: 164);
(A) an allele wherein the base at the position corresponding to position 201 of SEQ ID NO: 1 is A (e.g. an allele comprising the nucleotide sequence of SEQ ID NO: 69);
(B-1) an allele wherein the base at the position corresponding to position 40 of SEQ ID NO: 2 is T (e.g. an allele comprising the nucleotide sequence of SEQ ID NO: 70);
(B-2) an allele wherein the base at the position corresponding to position 44 of SEQ ID NO: 3 is T (e.g. an allele comprising the nucleotide sequence of SEQ ID NO: 71);
(B-3) an allele wherein the base at the position corresponding to position 41 of SEQ ID NO: 4 is C (e.g. an allele comprising the nucleotide sequence of SEQ ID NO: 72);
(B-4) an allele wherein the portion corresponding to positions 55-72 of SEQ ID NO: 5 is deleted (e.g. an allele comprising the nucleotide sequence of SEQ ID NO: 73); and
(C) an allele wherein the base at the position corresponding to position 49 of SEQ ID NO: 6 is A (e.g. an allele comprising the nucleotide sequence of SEQ ID NO: 74); and/or
   by detecting the absence of an allele selected from the group consisting of:
(x) an allele wherein the base at the position corresponding to position 201 of SEQ ID NO: 150 is T (e.g. an allele comprising the nucleotide sequence of SEQ ID NO: 150);
(a) an allele wherein the base at the position corresponding to position 201 of SEQ ID NO: 1 is C (e.g. an allele comprising the nucleotide sequence of SEQ ID NO: 1);
(b-1) an allele wherein the base at the position corresponding to position 44 of SEQ ID NO: 2 is A (e.g. an allele comprising the nucleotide sequence of SEQ ID NO: 2);
(b-2) an allele wherein the base at the position corresponding to position 40 of SEQ ID NO: 3 is C (e.g. an allele comprising the nucleotide sequence of SEQ ID NO: 3);
(b-3) an allele wherein the base at the position corresponding to position 41 of SEQ ID NO: 4 is G (e.g. an allele comprising the nucleotide sequence of SEQ ID NO: 4);
(b-4) an allele wherein the portion corresponding to positions 55-72 of SEQ ID NO: 5 is not deleted (e.g. an allele comprising the nucleotide sequence of SEQ ID NO: 5); and
(c) an allele wherein the base at the position corresponding to position 49 of SEQ ID NO: 6 is C (e.g. an allele comprising the nucleotide sequence of SEQ ID NO: 6).

The absence of the genetic feature(s) of the present invention can be determined by detecting the absence of an allele selected from the group consisting of:
(X) an allele wherein the base at the position corresponding to position 201 of SEQ ID NO: 150 is C (e.g. an allele comprising the nucleotide sequence of SEQ ID NO: 164);
(A) an allele wherein the base at the position corresponding to position 201 of SEQ ID NO: 1 is A (e.g. an allele comprising the nucleotide sequence of SEQ ID NO: 69);
(B-1) an allele wherein the base at the position corresponding to position 40 of SEQ ID NO: 2 is T (e.g. an allele comprising the nucleotide sequence of SEQ ID NO: 70);
(B-2) an allele wherein the base at the position corresponding to position 44 of SEQ ID NO: 3 is T (e.g. an allele comprising the nucleotide sequence of SEQ ID NO: 71);
(B-3) an allele wherein the base at the position corresponding to position 41 of SEQ ID NO: 4 is C (e.g. an allele comprising the nucleotide sequence of SEQ ID NO: 72);
(B-4) an allele wherein the portion corresponding to positions 55-72 of SEQ ID NO: 5 is deleted (e.g. an allele comprising the nucleotide sequence of SEQ ID NO: 73); and
(C) an allele wherein the base at the position corresponding to position 49 of SEQ ID NO: 6 is A (e.g. an allele comprising the nucleotide sequence of SEQ ID NO: 74); and/or
   by detecting the presence of an allele selected from the group consisting of:
(x) an allele wherein the base at the position corresponding to position 201 of SEQ ID NO: 150 is T (e.g. an allele comprising the nucleotide sequence of SEQ ID NO: 150);
(a) an allele wherein the base at the position corresponding to position 201 of SEQ ID NO: 1 is C (e.g. an allele comprising the nucleotide sequence of SEQ ID NO: 1);
(b-1) an allele wherein the base at the position corresponding to position 44 of SEQ ID NO: 2 is A (e.g. an allele comprising the nucleotide sequence of SEQ ID NO: 2);
(b-2) an allele wherein the base at the position corresponding to position 40 of SEQ ID NO: 3 is C (e.g. an allele comprising the nucleotide sequence of SEQ ID NO: 3);
(b-3) an allele wherein the base at the position corresponding to position 41 of SEQ ID NO: 4 is G (e.g. an allele comprising the nucleotide sequence of SEQ ID NO: 4);
(b-4) an allele wherein the portion corresponding to positions 55-72 of SEQ ID NO: 5 is not deleted (e.g. an allele comprising the nucleotide sequence of SEQ ID NO: 5); and
(c) an allele wherein the base at the position corresponding to position 49 of SEQ ID NO: 6 is C (e.g. an allele comprising the nucleotide sequence of SEQ ID NO: 6).

Specific examples of methods of detecting the genetic features of the present invention include, but not limited to, PCR method, TaqMan PCR method, sequencing method, microarray method, Invader method, TILLING method, RAD method, RFLP method, PCR-SSCP method, AFLP method, SSLP method, CAPS method, dCAPS method, ASO method, ARMS method, DGGE method, CCM method, DOL method, MALDI-TOF/MS method, TDI method, padlock probe method, molecular beacon method, DASH method, UCAN method, ECA method, PINPOINT method, PROBE method, VSET method, Survivor assay, Sniper assay, Luminex assay, GOOD method, LCx method, SNaPshot method, Mass ARRAY method, pyrosequencing method, SNP-IT method, melting curve analysis method, etc.

In the case of PCR method, it is preferable to generate a primer such that the 3' end portion has a sequence complementary to the polymorphic site of the present invention. By using a primer designed in this way, the polymerase extension reaction proceeds because the primer hybridizes completely to the template if the template sample has the polymorphism, whereas if the template does not have the variation of the present invention, the extension reaction does not occur because the nucleotide at the 3' end of the primer mismatches the template. Therefore, PCR amplification is performed using such a primer, and the amplification product is analyzed by agarose gel electrophoresis or the like, and if an amplification product of a predetermined size can be confirmed, the template as the sample has a variation, and if the amplification product is not present, it can be judged that the template does not have a variation.

Alternatively, the genetic feature(s) of the present invention can be detected by designing the primer sequence so that the polymorphism of the present invention and the primer sequence do not overlap and the genetic variation of the present invention can be PCR amplified, and by sequencing the nucleotide sequence of the amplified nucleotide fragment.

For PCR and agarose gel electrophoresis see Sambrook, Fritsch and Maniatis, "Molecular Cloning: A Laboratory Manual" 2nd Edition (1989), Cold Spring Harbor Laboratory Press.

TaqMan PCR method uses fluorescently labeled allele-specific oligos and Taq DNA polymerases (Livak, K. J. Genet). Anal. 14, 143 (1999); Morris T. et al., J. Clin. Microbiol. 34, 2933 (1996)).

The sequencing method is a method of analyzing the presence or absence of a variation by amplifying a region containing the variation by PCR and sequencing the DNA sequence using a Dye Terminator or the like (Sambrook, Fritsch and Maniatis, "Molecular Cloning: A Laboratory Manual" 2nd Edition (1989), Cold Spring Harbor Laboratory Press).

A DNA microarray is one in which one end of a nucleotide probe is immobilized in an array on a support, and includes a DNA chip, a Gene chip, a microchip, a bead array, and the like. By using a probe containing a sequence complementary to the polymorphism of the present invention, the presence or absence of the polymorphism of the present invention can be comprehensively detected. DNA microarray assays such as DNA chips include GeneChip assays (see Affymetrix; U.S. Pat. Nos. 6,045,996; 5,925,525; and 5,858,659). The GeneChip technique utilizes a miniaturized, high density microarray of oligonucleotide probes affixed to a chip.

The invader method combines the hybridization of two reporter probes specific for each allele of a polymorphism such as SNPs and one invader probe to template DNA and the cleavage of DNA by Cleavase enzyme with a special endonuclease activity which cleaves a DNA by recognizing its structure (Livak, K. J. Biomol. Eng. 14, 143-149 (1999); Morris T. et al., J. Clin. Microbiol. 34, 2933 (1996); Lyamichev, V. et al., Science, 260, 778-783 (1993), and the like).

TILLING (Targeting Induced Local Lesions IN Genomes) method is a method in which mutational mismatches in the genomes of a mutagenized mutant population are screened by PCR-amplification and CEL I nuclease-treatment.

In one embodiment, the genetic feature X of the present invention can be detected, without limitations, by CAPS method using a primer set that can amplify a region comprising a sequence shown in any of SEQ ID NOs: 153 to 155, and a restriction enzyme (e.g. Hpy188I) that cleaves the polynucleotides of SEQ ID NOs: 153 to 155 but does not cleave the polynucleotides of SEQ ID NOs: 165 to 167 or a restriction enzyme that does not cleave the polynucleotides of SEQ ID NOs: 153 to 155 but cleaves the polynucleotides of SEQ ID NOs: 165 to 167. Non-limiting examples of the primer set include the following.
Forward primer: GTCAACATCATCCAGTTTGAAGCAC (SEQ ID NO: 156)
Reverse primer: GAACTGCCTTCATATCATGGATCCA (SEQ ID NO: 157)

In one embodiment, the genetic feature A of the present invention can be detected, without limitations, by CAPS method using a primer set that can amplify a region comprising a sequence shown in any of SEQ ID NOs: 19 to 21, and a restriction enzyme that cleaves the polynucleotides of SEQ ID NOs: 19 to 21 but does not cleave the polynucleotides of SEQ ID NOs: 75 to 77 or a restriction enzyme (e.g. Hpy188I) that does not cleave the polynucleotides of SEQ ID NOs: 19 to 21 but cleaves the polynucleotides of SEQ ID NOs: 75 to 77. Non-limiting examples of the primer set include the following.
Forward primer: ATGGTTTGGGAATAGCTCTGTTGTT (SEQ ID NO: 37)
Reverse primer: AGAACTTTGTTCTTGAACCTCTTG (SEQ ID NO: 38)

In one embodiment, the genetic feature B of the present invention can be detected, without limitations, by dCAPS method or the like using the following primer set and a restriction enzyme.
(B-1) a primer set comprising a forward primer comprising the nucleotide sequence shown in SEQ ID NO: 45 and a reverse primer comprising the nucleotide sequence shown in SEQ ID NO: 46;
(B-2) a primer set comprising a forward primer comprising the nucleotide sequence shown in SEQ ID NO: 50 and a reverse primer comprising the nucleotide sequence shown in SEQ ID NO: 51;
(B-3) a primer set comprising a forward primer comprising the nucleotide sequence shown in SEQ ID NO: 55 and a reverse primer comprising the nucleotide sequence shown in SEQ ID NO: 56; and
(B-4) a primer set comprising a forward primer comprising the nucleotide sequence shown in SEQ ID NO: 60 and a reverse primer comprising the nucleotide sequence shown in SEQ ID NO: 61.

However, the primer set is not limited to those having the sequences of SEQ ID NOs: 45, 46, 50, 51, 55, 56, 60 or 61. For example, the forward primer can have in its 3' end a sequence from the 3' end of SEQ ID NO: 45, 50, 55 or 60 to 15 bases upstream thereof (see the table below), and the reverse primer can have in its 3' end a sequence from the 3' end of SEQ ID NO: 46, 51, 56 or 61 to 15 bases upstream thereof (see the table below). Such a primer may be 15 to 50 bases long or 20 to 45 bases long.

**Table 1 Examples of primer sets**

| Genetic feature (primer set name) | Forward primer (sequence from the 3' end to 15 bases upstream thereof) | Reverse primer (sequence from the 3' end to 15 bases upstream thereof) |
|---|---|---|
| B-1 (B-1') | 5'-CAAACAACCGGGTAC-3' (SEQ ID NO: 78) | 5'-AGACATTGGCAACTC-3' (SEQ ID NO: 79) |
| B-2 (B-2') | 5'-ATTTATTGTATCTAG-3' (SEQ ID NO: 80) | 5'-GTACACATGCTACAC-3' (SEQ ID NO: 81) |
| B-3 (B-3') | 5'-ACGAAACCCGCTTAA-3' (SEQ ID NO: 82) | 5'- TAATCCTTGAATTAG-3' (SEQ ID NO: 83) |
| B-4 (B-4') | 5'-ACACGTATACTAATC-3' (SEQ ID NO: 84) | 5'-CATGGTATGTACAAC-3' (SEQ ID NO: 85) |

The primer set is not limited to those having the sequences of SEQ ID NOs: 45, 46, 50, 51, 55, 56, 60 or 61. For example, the forward primer can have or comprise a sequence of any 15 or more consecutive bases in SEQ ID NO: 45, 50, 55 or 60, and the reverse primer can have or comprise a sequence of any 15 or more consecutive bases in SEQ ID NO: 46, 51, 56 or 61.
(B-1") A primer set comprising a forward primer having or comprising a sequence of any 15 or more consecutive bases in SEQ ID NO: 45 and a reverse primer having or comprising a sequence of any 15 or more consecutive bases in SEQ ID NO: 46;
(B-2") a primer set comprising a forward primer having or comprising a sequence of any 15 or more consecutive bases in SEQ ID NO: 50 and a reverse primer having or comprising a sequence of any 15 or more consecutive bases in SEQ ID NO: 51;
(B-3") a primer set comprising a forward primer having or comprising a sequence of any 15 or more consecutive bases in SEQ ID NO: 55 and a reverse primer having or comprising a sequence of any 15 or more consecutive bases in SEQ ID NO: 56; or
(B-4") a primer set comprising a forward primer having or comprising a sequence of any 15 or more consecutive bases in SEQ ID NO: 60 and a reverse primer having or comprising a sequence of any 15 or more consecutive bases in SEQ ID NO: 61.

Such a primer may be 15 to 50 bases long, 20 to 45 bases long, or 30 to 65 bases long as long as the arbitrary sequence of 15 or more consecutive bases is present at the 3' end.

Examples of the restriction enzymes to be combined with the above primers include the following.

**Table 2 Restriction enzymes to be combined with primer**

| Primer | Restriction enzyme |
|---|---|
| (B-1), (B-1'), (B-1") | Kpnl |
| (B-2), (B-2'), (B-2") | Xbal |
| (B-3), (B-3'), (B-3") | AflII |

In one embodiment, the genetic feature C of the present invention can be detected by dCAPS method using the following primer set and a restriction enzyme.

### Primer set:

A primer set comprising a forward primer comprising a sequence which is positioned at the 3' end and selected from SEQ ID NOs: 86 to 109, and an optional sequence which is added to the 5' end of the sequence and is of any consecutive upstream bases following position 28 of SEQ ID NO: 6 (e.g. a consecutive sequence of any length), and a reverse primer comprising a sequence (e.g. SEQ ID NO: 65 or 110) complementary to a sequence of any consecutive 20 bases or more which is positioned downstream of position 50 of SEQ ID NO: 6. The sequences of the primers can be optimized within a range that satisfies the conditions described above. For the optimization of primer design, see, for example, Sambrook and Russell, "Molecular Cloning: A Laboratory Manual" 3rd Edition (2001), Cold Spring Harbor Laboratory Press. Each of the primers may be 15 to 50 base long, 18 to 48 base long, 20 to 45 base long, 30 to 65 base long, or the like.

### Restriction enzyme:

A restriction enzyme appropriate for each of SEQ ID NOs: 86 to 109 is shown below. In the sequences described below, "R" represents A or G, and "Y" represents C or T.

**Table 3 Restriction enzymes appropriate for sequence contained in forward primer**

| Sequence contained in forward primer | Restriction enzyme |
|---|---|
| TTCAGGTAATAAAAGGCCTT (SEQ ID NO: 86) | Ddel |
| TTCAGGTAATAAAAGGCACT (SEQ ID NO: 87) | MaeI/SpeI |
| TTCAGGTAATAAAAGGCTTA (SEQ ID NO: 88) | AflII /Msel |
| TTCAGGTAATAAAAGGCTTG (SEQ ID NO: 89) | Bce83I |
| TTCAGGTAATAAAAGGCCTC (SEQ ID NO: 90) | BseMII |
| TTCAGGTAATAAAAGGCACG (SEQ ID NO: 91) | Bsil |
| TTCAGGTAATAAAAGTCATG (SEQ ID NO: 92) | BspHI /Hpy178III |
| TTCAGGTAATAAAAGGCTRT (SEQ ID NO: 93) | Sfel |
| TTCAGGTAATAAAAGGCTTR (SEQ ID NO: 94) | SmlI |
| TTCAGGTAATAAAAGGCAGC (SEQ ID NO: 95) | EcoP15I |
| TTCAGGTAATAAAAGGCYCG (SEQ ID NO: 96) | Aval |
| TTCAGGTAATAAAAGTGATC (SEQ ID NO: 97) | BclI |
| TTCAGGTAATAAAAGGGAGG (SEQ ID NO: 98) | BseRI |
| TTCAGGTAATAAAAGGCTGC (SEQ ID NO: 99) | CviRI /PstI |
| TTCAGGTAATAAAAGGAACC (SEQ ID NO: 100) | DrdII |
| TTCAGGTAATAAAAGGCTGA (SEQ ID NO: 101) | Eco57I |
| TTCAGGTAATAAAAGGCTGG (SEQ ID NO: 102) | Gsul |
| TTCAGGTAATAAAAGGGGTG (SEQ ID NO: 103) | HphI |
| TTCAGGTAATAAAAGGTCTG (SEQ ID NO: 104) | Hpyl881 |
| TTCAGGTAATAAAAGGGAAG (SEQ ID NO: 105) | MboII |
| TTCAGGTAATAAAAGGTCGT (SEQ ID NO: 106) | Pfl11081 |
| TTCAGGTAATAAAAGTTATA (SEQ ID NO: 107) | Psil |
| TTCAGGTAATAAAAGGCTCG (SEQ ID NO: 108) | TaqI /XhoI |
| TTCAGGCGATAAAAGGCGTT (SEQ ID NO: 109) | StySKI |

In a specific embodiment, the genetic feature C of the present invention can be detected by dCAPS method using the following primer set and restriction enzyme.

**Table 4 Combination of primer set and restriction enzyme**

| Sequence of forward primer | Sequence of reverse primer | Restriction enzyme |
|---|---|---|
| SEQ ID NO: 111 | SEQ ID NO: 65 | Ddel |
| SEQ ID NO: 112 | SEQ ID NO: 65 | MaeI/SpeI |
| SEQ ID NO: 113 | SEQ ID NO: 65 | AflII /Msel |
| SEQ ID NO: 114 | SEQ ID NO: 65 | Bce83I |
| SEQ ID NO: 115 | SEQ ID NO: 65 | BseMII |
| SEQ ID NO: 116 | SEQ ID NO: 65 | Bsil |
| SEQ ID NO: 117 | SEQ ID NO: 65 | BspHI /Hpy 178III |
| SEQ ID NO: 118 | SEQ ID NO: 65 | Sfel |
| SEQ ID NO: 119 | SEQ ID NO: 65 | SmlI |
| SEQ ID NO: 120 | SEQ ID NO: 65 | EcoP15I |
| SEQ ID NO: 121 | SEQ ID NO: 65 | Aval |
| SEQ ID NO: 122 | SEQ ID NO: 65 | BclI |
| SEQ ID NO: 123 | SEQ ID NO: 65 | BseRI |
| SEQ ID NO: 124 | SEQ ID NO: 65 | CviRI /PstI |
| SEQ ID NO: 125 | SEQ ID NO: 65 | DrdII |
| SEQ ID NO: 126 | SEQ ID NO: 65 | Eco57I |
| SEQ ID NO: 127 | SEQ ID NO: 65 | Gsul |
| SEQ ID NO: 128 | SEQ ID NO: 65 | HphI |
| SEQ ID NO: 129 | SEQ ID NO: 65 | Hpy188I |
| SEQ ID NO: 130 | SEQ ID NO: 65 | MboII |
| SEQ ID NO: 131 | SEQ ID NO: 65 | Pfl1108I |
| SEQ ID NO: 132 | SEQ ID NO: 65 | Psil |
| SEQ ID NO: 133 | SEQ ID NO: 65 | TaqI /XhoI |
| SEQ ID NO: 134 | SEQ ID NO: 65 | StySKI |

The screening methods of the present invention may further comprise a step of determining the tyramine content of a tissue (e.g. leaf) of the test stevia plant tissue for which the genetic features of the present invention have been detected. The determination of the tyramine content is as described in the section relating to the plant of the present invention. In this embodiment, the screening method of the present invention may be applied to daughter plants obtained by selecting individuals with a lower content of tyramine from among the test stevia plants in which the genetic feature(s) of the present invention is/are detected, and crossing the selected individuals with another stevia plants. Thus, the screening method of the present invention may comprise one or more of the following steps.
(i) Detecting the genetic feature(s) of the present invention from the genome of a test stevia plant;
(ii) determining the tyramine content of the test stevia plant tissue in which the genetic feature(s) has/have been detected;
(iii) selecting an individual with a lower content of tyramine from among the test stevia plants in which the genetic feature(s) of the present invention has/have been detected;
(iv) crossing the selected individual with a lower content of tyramine with another stevia plant;
(v) detecting the genetic feature(s) of the present invention from the genome of daughter plants obtained by crossing,
(vi) measuring the tyramine content of the tissue of the daughter plants in which the genetic feature(s) has/have been detected,
(vii) selecting individuals having a lower tyramine content from among the daughter plants in which the genetic features are detected.

Individuals with a low content of tyramine of choice may be, for example, up to 50%, up to 40%, up to 30%, up to 20%, up to 10%, up to 5%, up to 4%, up to 3%, up to 2%, or up to 1% of the test stevia plants in which the genetic feature(s) of the present invention has/have been detected, with respect to the low content of tyramine. Other stevia plants to be crossed may or may not contain the genetic feature(s) of the present invention. In the above embodiment, steps (iv) to (vii) can be repeated a plurality of times. In this way, stevia plants with a lower content of tyramine can be screened.

In the screening method of the present invention, the test stevia plant may be a natural plant or a non-transgenic plant. Non-transgenic plants are as described in the section relating to the plant of the present invention.

In the screening method of the present invention, the test stevia plant may include a stevia plant subjected to a mutagenesis treatment and a progeny plant thereof. The mutagenesis treatment is as described in the section relating to the plant of the present invention, and includes treatment with a mutagen, treatment with radiation or irradiation with light, and the like.

The present invention also provides the above-mentioned primer set, e.g. the primer set comprising the forward primer of SEQ ID NO: 156 and the reverse primer of SEQ ID NO: 157, the primer set comprising the forward primer of SEQ ID NO: 37 and the reverse primer of SEQ ID NO: 38, any one or more primer set(s) selected from the group consisting of the primer sets (B-1) to (B-4), (B-1') to (B-4') and (B-1") to (B-4") above, and/or the primer set described in Tables 2 and 3 above. The present invention further provides a primer set capable of amplifying a region having a nucleotide sequence selected from the group consisting of SEQ ID NOs: 150, 164, 1 to 6, 69 to 74 by PCR, for example, a primer set with a forward primer comprising a nucleotide sequence of SEQ ID NO: 151 and a reverse primer comprising a nucleotide sequence of SEQ ID NO: 152, a primer set with a forward primer comprising a nucleotide sequence of SEQ ID NO: 7 and a reverse primer comprising a nucleotide sequence of SEQ ID NO: 8, a primer set with a forward primer comprising a nucleotide sequence of SEQ ID NO: 9, and a reverse primer comprising a nucleotide sequence of SEQ ID NO: 10, a primer set with a forward primer comprising a nucleotide sequence of SEQ ID NO: 11 and a reverse primer comprising a nucleotide sequence of SEQ ID NO: 12, a primer set with a forward primer comprising a nucleotide sequence of SEQ ID NO: 13 and a reverse primer comprising a nucleotide sequence of SEQ ID NO: 14, a primer set with a forward primer comprising a nucleotide sequence of SEQ ID NO: 15 and a reverse primer comprising a nucleotide sequence of SEQ ID NO: 16, and a primer set with a forward primer comprising a nucleotide sequence of SEQ ID NO: 17 and a reverse primer comprising a nucleotide sequence of SEQ ID NO: 18.

In addition, the present invention provides a probe capable of detecting the presence and/or absence of the genetic features of the present invention, which may be referred to as the "probe of the present invention" hereinafter. The probe of the present invention may have a structure suitable for various detection methods for the presence and/or absence of the genetic feature(s) of the present invention. For example, the probe of the present invention may comprise a nucleotide sequence complementary to a portion of a genome comprising a variation site of the present invention. Non-limiting examples of such probes include those comprising a nucleotide sequence selected from SEQ ID NOs: 153 to 155, 165 to 167, 19 to 36, 75 to 77, 135 to 149. Of these sequences, SEQ ID NOs: 153 to 155, 19 to 36 are specific for alleles comprising the variation of the present invention, and SEQ ID NOs: 165 to 167, 75 to 77, 135 to 149 are specific for alleles not containing the variation of the present invention. The presence of the genetic feature(s) of the present invention may be detected by detection of an allele comprising the variation(s) of the present invention and/or by non-detection of an allele not comprising the variation(s) of the present invention, and the absence of the genetic feature(s) of the invention by non-detection of an allele comprising the variation(s) of the present invention and/or by detection of an allele not comprising the variation(s) of the present invention. The probes of the present invention preferably have a label. Non-limiting examples of such labels include fluorescent labels, luminescent labels, radioactive labels, dyes, enzymes, quenchers, binding moieties with detectable labels, and the like. In a specific embodiment, the probe of the present invention has a nucleotide sequence selected from SEQ ID NOs: 153 to 155, 165 to 167, 19 to 36, 75 to 77, 135 to 149 and a label.

The present invention further provides a kit, for example, a kit for screening, comprising a primer set that can amplify a region comprising a sequence shown in any of SEQ ID NOs: 153 to 155 and 165 to 167, for example, a primer set comprising the combination of a forward primer comprising the nucleotide sequence of SEQ ID NO: 151 and a reverse primer comprising the nucleotide sequence of SEQ ID NO: 152, and a restriction enzyme (e.g. Hpy188I) that cleaves the polynucleotides of SEQ ID NOs: 153 to 155 but does not cleave the polynucleotides of SEQ ID NOs: 165 to 167 or a restriction enzyme that does not cleave the polynucleotides of SEQ ID NOs: 153 to 155 but cleaves the polynucleotides of SEQ ID NOs: 165 to 167.

The present invention further provides a kit, for example, a kit for screening, comprising a primer set that can amplify a region comprising a sequence shown in any of SEQ ID NOs: 19 to 21, for example, a primer set comprising the combination of a forward primer comprising the nucleotide sequence of SEQ ID NO: 7 and a reverse primer comprising the nucleotide sequence of SEQ ID NO: 8, and a restriction enzyme that cleaves the polynucleotides of SEQ ID NOs: 19 to 21 but does not cleave the polynucleotides of SEQ ID NOs: 75 to 77 or a restriction enzyme (e.g. Hpy188I) that does not cleave the polynucleotides of SEQ ID NOs: 19 to 21 but cleaves the polynucleotides of SEQ ID NOs: 75 to 77.

The present invention further provides a kit, for example, a kit for screening comprising any one or more primer set(s) selected from the group consisting of the primer sets (B-1) to (B-4), (B-1') to (B-4') and (B-1") to (B-4"), and optionally a restriction enzyme.

In the kit, the restriction enzyme contained in the kit is Kpnl in the case of using any one or more primer set(s) selected from the group consisting of the primer sets (B-1), (B-1') and (B-1").

In the kit, the restriction enzyme contained in the kit is Xbal in the case of using any one or more primer set(s) selected from the group consisting of the primer sets (B-2), (B-2') and (B-2").

In the kit, the restriction enzyme contained in the kit is AflII in the case of using any one or more primer set(s) selected from the group consisting of the primer sets (B-3), (B-3') and (B-3").

The present invention further provides a kit, for example, a kit for screening, comprising a primer set comprising the combination of a forward primer and a reverse primer shown in Tables 2 and 3 above, and a restriction enzyme appropriate therefor.

In another embodiment of the kit:
in case the primer set comprises a forward primer having or comprising a sequence of any consecutive 15 bases or more in SEQ ID NO: 45, the restriction enzyme comprises Kpnl;
in case the primer set comprises a forward primer having or comprising a sequence of any contiguous 15 bases or more in SEQ ID NO: 50, the restriction enzyme comprises Xbal; and
in case the primer set comprises a forward primer having or comprising a sequence of any consecutive 15 bases or more in SEQ ID NO: 55, the restriction enzyme comprises AflII.

The present invention also provides a screening kit comprising a primer set capable of amplifying by PCR a region having a nucleotide sequence selected from the group consisting of SEQ ID NOs: 153 to 155, 165 to 167, 1 to 6, 69 to 74, and a probe of the present invention.

These primer sets, probes and kits can be used to detect the genetic feature(s) of the present invention, used in the screening methods of the present invention, and the like. These primer sets and kits may also comprise an instruction including an explanation on the detection of genetic feature(s) of the present invention and on the screening method of the present invention, e.g. a written instruction, and media, e.g. a flexible disk, a CD, a DVD, a Blu-ray disk, a memory card, a USB memory, etc., having recorded thereon information regarding the method of use.

### 4. Method of producing plant-derived extract, and product comprising the extract

In a further aspect, the present invention provides a method of producing a low tyramine-content extract, comprising a step of obtaining an extract from the plant of the present invention, or a seed or a leaf (e.g. dried leaf or fresh leaf) of the plant (hereinafter, may be referred to as the "extract production method of the present invention"). The present invention further provides a method of producing a steviol glycoside purified product, comprising a step of purifying steviol glycosides from an extract obtained by the extract production method of the present invention (hereinafter, may be referred to as the "steviol glycoside purified product production method of the present invention").

Specifically, the present invention provides a method of producing a steviol glycoside purified product, comprising a step of obtaining an extract containing steviol glycosides from the low tyramine-content stevia plant of the present invention, the low tyramine-content stevia plant screened for by the screening method of the present invention, or the low tyramine-content stevia plant produced by the method of the present invention, and a step of purifying steviol glycosides from the obtained extract.

The extract containing steviol glycosides can be obtained by reacting a fresh leaf or dried leaf of the plant of the present invention with a suitable solvent (an aqueous solvent such as water or an organic solvent such as an alcohol, ether or acetone). For the extraction conditions, etc., see a method described in Ohta et al., J. Appl. Glycosci., Vol. 57, No. 3 (2010) or WO2010/038911, or a method described in Examples mentioned later.

Individual steviol glycosides can be purified from the extract containing steviol glycosides by use of a method known in the art such as a gradient of ethyl acetate or any of other organic solvents:water, high performance liquid chromatography (HPLC), gas chromatography, time-of-flight mass spectrometry (TOF-MS), or ultra (high) performance liquid chromatography (UPLC).

Examples of the steviol glycosides include RebA, RebB, RebC, RebD, RebE, RebF, Rebl, RebJ, RebK, RebM, RebN, RebO, RebQ, RebR, dulcoside A, rubusoside, steviol, steviolmonoside, steviolbioside, and stevioside. In one embodiment, the steviol glycosides include RebA, RebB, RebC, RebD, RebE, RebF, RebM, RebN, RebO, stevioside, steviolbioside, rubusoside, dulcoside A or a combination thereof.

The extract obtained by the extract production method of the present invention (hereinafter, may be referred to as the "extract of the present invention") comprises tyramine at lower content as compared with the wild type stevia species.

The extract of the present invention may comprise tyramine at a content of 95% or less, 90% or less, 85% or less, 80% or less, 75% or less, 70% or less, 65% or less, 60% or less, 55% or less, 50% or less, 45% or less, 40% or less, 35% or less, 30% or less, 25% or less, 20% or less, 15% or less, 10% or less, 5% or less, 4% or less, 3% or less, 2% or less, 1% or less, 0.5% or less, 0.3% or less, 0.1% or less, 0.05% or less, 0.03% or less, 0.001% or less, or the like as compared with an extract obtained from the wild type stevia species. The extract of the present invention and the extract obtained from the wild type stevia species may be those obtained by the same process.

The extract of the present invention thus obtained and/or the steviol glycoside purified product obtained by the method of producing a steviol glycoside purified product according to the present invention can be mixed with other component(s) to produce a novel medicament, flavor or food or beverage containing steviol glycosides with low tyramine content. Accordingly, in an alternative aspect, the present invention provides a method of producing a medicament, a flavor or a food or beverage, comprising a step of mixing the extract of the present invention and/or the steviol glycoside purified product obtained by the method of producing a steviol glycoside purified product according to the present invention with other component(s). The present invention further provides a novel medicament, flavor or food or beverage containing steviol glycosides with low tyramine content, obtained by the production method. In this context, the food or beverage means a drink and a food. Thus, in a certain embodiment, the present invention provides a novel medicament, flavor, drink or food and also provides a method of producing the medicament, the flavor, the drink or the food. The medicament, the flavor or the food or beverage of the present invention with low tyramine content is particularly useful for subjects receiving a monoamine oxidase inhibitor.

### 5. Nucleotide sequence relating to plants of the present invention

In another aspect, the present invention provides nucleotide sequences relating to the plant of the present invention. Specific embodiments of the nucleotide sequences relating to the plant of the present invention having the genetic feature X of the present invention comprise or consist of a nucleotide sequence selected from SEQ ID NOs: 164 to 167. Specific embodiments of the nucleotide sequences relating to the plant of the present invention having the genetic feature X and the genetic feature A of the present invention comprise a nucleotide sequence selected from SEQ ID NOs: 164 to 167 and a nucleotide sequence selected from SEQ ID NOs: 19 to 21 and 69. Specific embodiments of the nucleotide sequences relating to the plant of the present invention having the genetic feature X and the genetic feature B of the present invention (i.e. at least one of the genetic features B-1 to B-4 of the present invention)comprise a nucleotide sequence selected from SEQ ID NOs: 164 to 167 and a nucleotide sequence selected from SEQ ID NOs: 22 to 33 and 70 to 73. Specific embodiments of the nucleotide sequences relating to the plant of the present invention having the genetic feature X and the genetic feature C of the present invention comprise a nucleotide sequence selected from SEQ ID NOs: 164 to 167 and a nucleotide sequence selected from SEQ ID NOs: 34 to 36 and 74. Specific embodiments of the nucleotide sequences relating to the plant of the present invention having the genetic feature X, the genetic feature A and the genetic feature B of the present invention comprise a nucleotide sequence selected from SEQ ID NOs: 164 to 167, a nucleotide sequence selected from SEQ ID NOs: 19 to 21 and 69 and a nucleotide sequence selected from SEQ ID NOs: 22 to 33 and 70 to 73. Specific embodiments of the nucleotide sequences relating to the plant of the present invention having the genetic feature X, the genetic feature A and the genetic feature C of the present invention comprise a nucleotide sequence selected from SEQ ID NOs: 164 to 167, a nucleotide sequence selected from SEQ ID NOs: 19 to 21 and 69 and a nucleotide sequence selected from SEQ ID NOs: 34 to 36 and 74. Specific embodiments of the nucleotide sequences relating to the plant of the present invention having the genetic feature X, the genetic feature B and the genetic feature C of the present invention comprise a nucleotide sequence selected from SEQ ID NOs: 164 to 167, a nucleotide sequence selected from SEQ ID NOs: 22 to 33 and 70 to 73 and a nucleotide sequence selected from SEQ ID NOs: 34 to 36 and 74. Specific embodiments of the nucleotide sequences relating to the plant of the present invention having all the genetic features X and A to C of the present invention comprise a nucleotide sequence selected from SEQ ID NOs: 164 to 167, a nucleotide sequence selected from SEQ ID NOs: 19 to 21 and 69, a nucleotide sequence selected from SEQ ID NOs: 22 to 33 and 70 to 73 and a nucleotide sequence selected from SEQ ID NOs: 34 to 36 and 74.

### EXAMPLES

Hereinafter, the present invention will be described with reference to Experimental Examples, Examples, etc. However, the present invention is not limited by these specific embodiments.

### Example 1 - Measurement of tyramine and RebD contents

### (1) Measurement of tyramine content

5 g of fresh leaves sampled from each individual was collected into a homogenizer cup, to which 10 mL of 20% trichloroacetic acid and 50 mL of water were then added, and the mixture was homogenized at 15000 rpm for 10 minutes. This homogenate was brought to a constant volume of 200 mL with water, mixed, left for 30 minutes, and then centrifuged and filtered, if necessary, to prepare a test solution. A 5 mL aliquot was separated from the test solution, and 5 mL of 0.1 mol/L sodium octanesulfonate was added thereto and well mixed, followed by loading to C18, 1000 mg minicolumn (Sep-Pak(R) Vac 6cc 1g C18 Cartridges, Waters Corp.). Subsequently, the minicolumn was washed with 20 mL of water, followed by the elution of amines with 10 mL of methanol:water (8:2). The whole amount of the eluate was collected and concentrated under reduced pressure at 40°C or lower to prepare approximately 1 mL of a concentrate. 0.5 mL of an internal standard solution (solution of 1,8-diaminooctane at a concentration of 20 µg/mL in 0.5 mol/L hydrochloric acid), 0.2 g of anhydrous sodium carbonate, and 2 mL of a 1% solution of dansyl chloride in acetone were added to this concentrate and well mixed, and the mixture was warmed in a water bath of 45°C for 1 hour with light shielded. After the warming, 0.5 mL of a 10% proline solution was added thereto, shaken, and left for 10 minutes. Then, 5 mL of toluene was added thereto and vigorously shaken for 1 minute, and the toluene layer was collected as an upper layer. Toluene was added thereto again, and this operation was performed twice. The toluene layers were combined and concentrated into dryness under reduced pressure at 40°C or lower. 1 mL of acetonitrile was added to the residue and well mixed to prepare a sample for HPLC analysis.

The HPLC analysis was conducted under the following conditions.

**Table 5 HPLC analysis conditions**

| HPLC | LC-10 (Shimadzu Corp.) |
|---|---|
| Column | Cosmosil ^{(R)} 5C18-AR-II (length 25 cm, inside diameter 4.6 mm, particle size 5 µm, Nacalai Tesque, Inc.) |
| Column temperature | 40°C |
| Mobile phase | Acetonitrile:water = 65:35 |
| Detector | Fluorescence detector (excitation wavelength: 325 nm, fluorescence wavelength: 525 nm) |
| Flow rate | 1 mL/min |
| Sample volume | 10 µL |

### (2) Measurement of RebD content

An appropriate amount of fresh leaves was sampled from each of the same individuals as those of (1), 0.25 g of fresh leaves was dried by freeze drying, and 0.05 g of homogenized dry matter thereof was added into pure water. Extraction by ultrasonic treatment for 20 minutes, and centrifugation and filtration were performed to obtain 0.33 mL of a liquid extract. The concentration of RebD was quantitatively determined by LC/MS-MS analysis on this liquid extract in LCMS8050 (Shimadzu Corp.).

### (3) Analysis results

The analysis results are shown in the table below.

**Table 6 Analysis results**

| Individual name | Tyramine content (%) | RebD content (%) |
|---|---|---|
| 1-1 | 0.030 | 4.135 |
| 2-1 | 0.030 | 3.900 |
| 3-1 | 0.037 | 0.856 |
| 4-1 | 0.041 | 4.007 |
| 5-1 | 0.041 | 2.215 |
| 6-1 | 0.058 | 1.842 |
| 7-1 | 0.092 | 0.938 |
| 2-2 | 0.096 | 0.985 |
| 8-1 | 0.163 | 0.854 |
| 2-3 | 0.171 | 1.395 |

The first number (on the left side of the hyphen) of each individual name represents the number of the parent (line) from which the individual was derived, and the number on the right side of the hyphen represents an individual number within the line to which the individual belonged. For example, individual name 1-1 and individual name 2-1 represent that these individuals were derived from different parents. Individual name 2-1 and individual name 2-2 represent that these were different individuals derived from the same parent (i.e. belonging to the same line). All the individuals are progeny of individuals genetically modified by mutagenesis treatment with ethylmethanesulfonic acid (EMS).

As a result of examining the presence or absence of the correlation of tyramine content with RebD content obtained by the analysis described above, negative correlation with a correlation coefficient of -0.615 was found between them. Figure 1 shows the results of analyzing the correlation of tyramine content with RebD content. When the presence or absence of significant difference was determined by the Welch's t test, there was significant difference with a p value of 0.0003 between the RebD content of an individual with a tyramine content of less than 0.037% and the RebD contents of the other individuals, a p value of 0.0454 between the RebD content of an individual with a tyramine content of less than 0.058% and the RebD contents of the other individuals, and a p value of 0.0239 between the RebD content of an individual with a tyramine content of less than 0.092% and the RebD contents of the other individuals.

### Example 2 - Detection of genetic features unique to low tyramine-content stevia plant

All of the individuals 2-1 to 2-3 tested in Example 1 were obtained by crossing male stocks (P1) having a high TSG-content genetic feature with female stocks (P2) having a high RebM-content genetic feature.

The high RebM-content genetic feature has at least one of the following features.

B-1: Homozygous for the allele wherein the base at the position corresponding to position 40 of SEQ ID NO: 2 is T.

B-2: Homozygous for the allele wherein the base at the position corresponding to position 44 of SEQ ID NO: 3 is T.

B-3: Homozygous for the allele wherein the base at the position corresponding to position 41 of SEQ ID NO: 4 is C.

B-4: Homozygous for the allele wherein the portion corresponding to positions 55-72 of SEQ ID NO: 5 is deleted.

The unpublished earlier application by the present applicant (PCT/JP2018/038064 filed on October 12, 2018) discloses that these genetic features are related to the high RebM-content characteristics.

The high TSG-content genetic feature has the following feature.

C: Heterozygous for the allele wherein the base at the position corresponding to position 49 of SEQ ID NO: 6 is A.

The unpublished earlier application by the present applicant (Japanese Patent Application No. 2018-144512 filed on July 31, 2018) discloses that the genetic feature is related to the high TSG-content characteristics.

In order to identify the genetic features of the individuals 2-1 to 2-3, genomic DNA was extracted from the fresh leaves of each of these individuals, and examined for the condition retaining the genetic features B-1 and C.

For the detection of genetic feature B-1, PCR was performed using the primers given below. A restriction enzyme (Kpnl) was added to the PCR product, and enzymatic reaction was performed at 37°C for treatment with the restriction enzyme. After the restriction enzyme treatment, electrophoresis was performed using a microchip type electrophoresis apparatus LabChip GX Touch HT (PerkinElmer). The marker was identified on the basis of a band pattern after the electrophoresis.

The primer sequences are as follows.
Forward primer:
   5'-TAATCATCCAAACCCTAATCTCGCCAAACAACCGGGTAC-3' (SEQ ID NO: 45)
Reverse primer:
   5'-GAGGAAGACATTGGCAACTC-3' (SEQ ID NO: 46)

When a restriction enzyme-treated product of approximately 260 bp (e.g. SEQ ID NO: 49) was not formed by the Kpnl restriction enzyme treatment of the obtained PCR product (approximately 297 bp long), the test subject was regarded as being positive for the genetic feature B-1.

For the detection of genetic feature C, PCR was performed using the primers given below. A restriction enzyme (Spel) was added to the PCR product, and enzymatic reaction was performed at 37°C for treatment with the restriction enzyme. After the restriction enzyme treatment, electrophoresis was performed using a microchip type electrophoresis apparatus LabChip GX Touch HT. The marker was identified on the basis of a band pattern after the electrophoresis.
Forward primer:
   5'-TTATTTAATGATCCAATGGAGGGGGTGATTCAGGTAATAAAAGGCACT-3' (SEQ ID NO: 64)
Reverse primer:
   5'-TGAGGGTTCTCAATTGATTTCCGATTGG-3' (SEQ ID NO: 65)

When a restriction enzyme-treated product of approximately 321 bp (e.g. SEQ ID NO: 68) was formed by the Spel restriction enzyme treatment of the obtained PCR product of approximately 367 bp (e.g. SEQ ID NO: 66 or 67), the test subject was regarded as being positive for the genetic feature C.

In order to further detect a marker for identifying individuals with low tyramine content, genomic DNA was extracted from the fresh leaves of each of the individuals 2-1 to 2-3 and sequenced with NGS (HiSeq 2500, Illumina, Inc.).

The results are shown in Table 6 below. In the table, a circle mark shows that the corresponding variation was detected, and an x mark shows that the corresponding variation was not detected. As shown in the results described below, individuals retaining genetic feature B-1 tended to have a lower tyramine content than that of the individuals not retaining genetic feature B-1. The following genetic features were detected only in the individual 2-1 with low tyramine content.

A: Homozygous for the allele wherein the base at the position corresponding to position 201 of SEQ ID NO: 1 is A.

X: Homozygous for the allele wherein the base at the position corresponding to position 201 of SEQ ID NO: 150 is C.

**Table 7**

| Individual number | Genetic feature | | | | Tyramine(*%*) | RebD (*%*) |
|---|---|---|---|---|---|---|
| | B-1 | C | A | X | | |
| 2-1 | ○ | ○ | ○ | ○ | 0.030 | 3.900 |
| 2-2 | ○ | ○ | × | × | 0.096 | 0.985 |
| 2-3 | × | ○ | × | × | 0.171 | 1.395 |

### INDUSTRIAL APPLICABILITY

The present invention enables the provision of a stevia extract and a steviol glycoside purified product with low tyramine content and can therefore provide a highly safe medicament, flavor or food or beverage, etc.

## Claims

1. A low tyramine-content stevia plant having a tyramine content of less than 0.092% per unit mass of dried leaf.

2. The plant according to claim 1, having a rebaudioside D content of 1% or more per unit mass of dried leaf.

3. The plant according to claim 1 or 2, wherein the plant is homozygous for the allele wherein the base at the position corresponding to position 201 of SEQ ID NO: 150 is C.

4. The plant according to any one of claims 1 to 3, wherein the plant is homozygous for the allele wherein the base at the position corresponding to position 201 of SEQ ID NO: 1 is A.

5. The plant according to any one of claims 1 to 4, further having at least one of the following genetic features.
(1) Homozygous for the allele wherein the base at the position corresponding to position 40 of SEQ ID NO: 2 is T.
(2) Homozygous for the allele wherein the base at the position corresponding to position 44 of SEQ ID NO: 3 is T.
(3) Homozygous for the allele wherein the base at the position corresponding to position 41 of SEQ ID NO: 4 is C.
(4) Homozygous for the allele wherein the portion corresponding to positions 55-72 of SEQ ID NO: 5 is deleted.

6. The plant according to any one of claims 1 to 5, wherein the plant is heterozygous for the allele wherein the base at the position corresponding to position 49 of SEQ ID NO: 6 is A.

7. The plant according to any one of claims 1 to 6, wherein the plant is a non-genetically modified plant.

8. The plant according to any one of claims 1 to 7, wherein the plant includes a stevia plant subjected to a mutagenesis treatment and a progeny plant thereof.

9. Seed, tissue, tissue culture or cell of a plant according to any one of claims 1 to 8.

10. Tissue, tissue culture or cell according to claim 9, which is selected from an embryo, a meristem cell, pollen, leaf, root, root apex, petal, protoplast, leaf section and callus.

11. A method of producing a low tyramine-content stevia plant having a tyramine content of less than 0.092% per unit mass of dried leaf, the method comprising a step of crossing a plant according to any one of claims 1 to 8 with a second stevia plant.

12. The method according to claim 11, wherein the second plant is a plant according to any one of claims 1 to 8.

13. An extract of a plant according to any one of claims 1 to 8, or of a seed, tissue, tissue culture or cell according to claim 9 or 10, wherein the extract has a low tyramine content.

14. A method of producing a low tyramine-content stevia extract, comprising a step of obtaining an extract from a plant according to any one of claims 1 to 8, or from a seed, tissue, tissue culture or cell according to claim 9 or 10.

15. A method of producing a low tyramine-content steviol glycoside purified product, comprising a step of purifying steviol glycosides from a stevia extract according to claim 13.

16. The method according to claim 15, wherein the steviol glycosides include rebaudioside A, rebaudioside B, rebaudioside C, rebaudioside D, rebaudioside E, rebaudioside F, rebaudioside M, rebaudioside N, rebaudioside O, stevioside, steviolbioside, rubusoside, dulcoside A or a combination thereof.

17. A low tyramine-content steviol glycoside purified product produced by a method according to claim 15 or 16.

18. A low tyramine-content food or beverage, sweetener composition, flavor or medicament comprising an extract of a tissue, tissue culture or cell according to claim 13, or a purified product according to claim 17.

19. A method of producing a food or beverage, a sweetener composition, a flavor or a medicament, comprising:
a step of providing an extract of a tissue, tissue culture or cell according to claim 13, or a purified product according to claim 17; and
a step of adding the extract or the purified product to a raw material for the food or beverage, sweetener composition, flavor or medicament.

20. A method of screening for a low tyramine-content stevia plant, comprising a step of detecting from the genome of a test stevia plant the presence and/or the absence of a genetic feature of being homozygous for the allele wherein the base at the position corresponding to position 201 of SEQ ID NO: 150 is C.

21. The method according to claim 20, further comprising a step of detecting from the genome of a test stevia plant the presence and/or the absence of a genetic feature of being homozygous for the allele wherein the base at the position corresponding to position 201 of SEQ ID NO: 1 is A.

22. The method according to claim 20 or 21, further comprising a step of detecting from the genome of a test stevia plant the presence and/or the absence of the following genetic features (1) to (4).
(1) Homozygous for the allele wherein the base at the position corresponding to position 40 of SEQ ID NO: 2 is T.
(2) Homozygous for the allele wherein the base at the position corresponding to position 44 of SEQ ID NO: 3 is T.
(3) Homozygous for the allele wherein the base at the position corresponding to position 41 of SEQ ID NO: 4 is C.
(4) Homozygous for the allele wherein the portion corresponding to positions 55-72 of SEQ ID NO: 5 is deleted.

23. The method according to any one of claims 20 to 22, further comprising a step of detecting from the genome of a test stevia plant the presence and/or the absence of a genetic feature of being heterozygous for the allele wherein the base at the position corresponding to position 49 of SEQ ID NO: 6 is A.

24. The method according to any one of claims 20 to 23, wherein the step of detecting a genetic feature is performed by use of CAPS method, dCAPS method or TaqMan PCR method.

25. The method according to any one of claims 20 to 24, further comprising a step of measuring the content of a tyramine in a test stevia plant tissue.

26. A screening kit for a low tyramine-content stevia plant, comprising a reagent for detecting the presence and/or the absence of a genetic feature of being homozygous for the allele wherein the base at the position corresponding to position 201 of SEQ ID NO: 150 is C.

27. The kit according to claim 26, further comprising a reagent for detecting the presence and/or the absence of a genetic feature of being homozygous for the allele wherein the base at the position corresponding to position 201 of SEQ ID NO: 1 is A.

28. The kit according to claim 26 or 27, further comprising a reagent for detecting the presence and/or the absence of the following genetic features (1) to (4).
(1) Homozygous for the allele wherein the base at the position corresponding to position 40 of SEQ ID NO: 2 is T.
(2) Homozygous for the allele wherein the base at the position corresponding to position 44 of SEQ ID NO: 3 is T.
(3) Homozygous for the allele wherein the base at the position corresponding to position 41 of SEQ ID NO: 4 is C.
(4) Homozygous for the allele wherein the portion corresponding to positions 55-72 of SEQ ID NO: 5 is deleted.

29. The kit according to any one of claims 26 to 28, further comprising a reagent for detecting the presence and/or the absence of a genetic feature of being heterozygous for the allele wherein the base at the position corresponding to position 49 of SEQ ID NO: 6 is A.

30. The kit according to any one of claims 26 to 29, wherein the reagent comprises a primer and/or a probe for use in CAPS method, dCAPS method or TaqMan PCR method.

31. A method of producing a low tyramine-content stevia plant, comprising a step of introducing a variation from T to C to a position corresponding to position 201 of SEQ ID NO: 150.

32. The method according to claim 31, wherein the introduction of the variation is performed by a mutagenesis treatment.
